# EUROPEAN PATENT APPLICATION

(11) **EP 4 060 346 A1**
(43) Date of publication of application: **21.09.2022**
(21) Application number: 20920371.0
(22) Date of filing: 26.06.2020
(51) Int. Cl.: G01N 33/68

(54) **COMPANION DIAGNOSIS BIOMARKER COMPOSITION AND COMPANION DIAGNOSIS KIT CONTAINING SAME**

(30) Priority: 18.02.2020 KR 20200019622; 11.05.2020 KR 20200056180
(71) Applicant: Innobation Bio Co., Ltd., Seoul 03929 (KR)
(72) Inventor: YOO, Byong Chul, Goyang-si Gyeonggi-do 10415 (KR); KIM, Kyung Hee, Seoul 03936 (KR); PARK, Jae Gwang, Goyang-si Gyeonggi-do 10364 (KR); WOO, Sang Myung, Seoul 06289 (KR); LEE, Young Ju, Seoul 03902 (KR); CHOI, Beom Kyu, Paju-si Gyeonggi-do 10893 (KR)
(74) Representative: J A Kemp LLP
(86) International application number: PCT/KR2020/008322
(87) International publication number: WO 2021/167177

(57) **Abstract**

The present invention relates to a companion diagnosis biomarker composition and a companion diagnosis kit containing the same and, particularly, to a companion diagnosis biomarker composition for predicting a therapeutic response to at least one immune checkpoint inhibitor from among a PD-1 immune checkpoint inhibitor and a PD-L1 immune checkpoint inhibitor, and a companion diagnosis kit containing the same. According to the present invention, there is an effect that it is possible to predict a therapeutic response to at least one immune checkpoint inhibitor from among a PD-1 immune checkpoint inhibitor and a PD-L1 immune checkpoint inhibitor not only through a companion diagnosis through cancer patient tissues, but also through proteomic analysis of cancer patient blood.

## Description

### [Technical Field]

The present invention relates to a companion diagnosis biomarker composition and a companion diagnosis kit containing the same and, particularly, to a companion diagnosis biomarker composition for predicting a therapeutic response to at least one immune checkpoint inhibitor from among a PD-1 immune checkpoint inhibitor and a PD-L1 immune checkpoint inhibitor and a companion diagnosis kit containing the same.

### [Background Art]

Companion diagnosis is a diagnostic technique for predicting the responsiveness of a patient to a specific drug treatment in advance. To overcome the disadvantages of most existing anticancer drugs that act on both cancer cells and normal cells to have large side effects, targeted anticancer drugs that selectively attack specific target proteins, immune anticancer drugs that activate the suppressed immune system of the human body to kill cancer cells, and the like have been developed.

However, since the targeted anticancer drug is effective only for cancer patients having a specific target protein even for the same type of cancer, therapeutic efficiency is very low unless patients with such a target protein are selected. Further, since the targeted anticancer drug depends on inhibition of cell growth and proliferation rather than cell apoptosis, there is a high possibility that resistance will develop due to continuous drug administration over a long period of time. Therefore, a group of patients who show an effect for a drug before administering the drug need to be selected through analysis of the target of an anticancer drug.

Immune anticancer drugs exhibit anticancer effects by enhancing the specificity, memory, and adaptiveness of the immune system. That is, since there are few side effects by precisely attacking only cancer cells using the immune system of the human body and the memory and adaptiveness of the immune system are used, patients who have an effect for immune anticancer drugs may have a sustained anticancer effect. However, for immune anticancer drugs, even in the case of the same type of cancer, the degree of anticancer effect differs depending on a specific patient, so companion diagnosis is required to predict the therapeutic response of patients through immune anticancer drugs in advance.

A companion diagnosis biomarker is used to screen a group of patients who show an effect in specific drug treatment, and here, a companion diagnosis biomarker can include proteins, DNA, RNA, metabolites, and the like as an index for predicting the responsiveness of a patient to a specific drug treatment in advance. That is, the companion diagnosis biomarker means a marker that can distinguish normal or pathological conditions, predict a therapeutic response, and objectively measure the therapeutic response in the case of a specific disease or cancer.

Meanwhile, as pharmaceutical companies reduce new drug development costs and the demand for targeted anticancer drugs is increased, the global market for companion diagnosis grows by 18% each year between 2013 and 2019, so that the size thereof is estimated to reach about $5.8 billion in 2019.

For example, Roche, which is one of the multinational pharmaceutical companies, has decided to start an anticancer drug treatment based on companion diagnosis by acquiring Genentech, which developed the first breast cancer-targeted anticancer drug "Herceptin" and its companion diagnosis kit "Herceptest".

Examples of a companion diagnosis kit include a method of confirming the overexpression of a specific protein through a immunohistochemical test, such as DAKO and HerpepTest, a method of confirming the gene amplification of a specific gene by a FISH or CISH test using a DNA probe, such as Ventana Medical Systems and INFORM HER-2/NEU, a method of testing and confirming the presence or absence of mutations in biomarker genes using a genomic techniques such as q-PCR, such as Roche Diagnostics and the cobas EGFR mutation test.

Meanwhile, immune anticancer drugs may be divided into passive immunotherapy and active immunotherapy, and examples of the passive immunotherapy include an immune checkpoint inhibitor, immune cell therapy, a therapeutic antibody, and the like. The immune checkpoint inhibitor is a drug that attacks cancer cells by blocking the activation of immune checkpoint proteins involved in the suppression of T cells to activate T cells, and examples thereof include CTLA-4, PD-1, PD-L1 immune checkpoint inhibitors, and the like.

The PD-1 immune checkpoint inhibitor is a second-generation immune anticancer drug, and Pembrolizumab (KEYTRUDA^{®}) developed by Merck & Co., Inc., and Nivolumab (OPDIVO^{®}) developed by Ono Pharmaceutical Co., Ltd. and BMS were approved by the US FDA in 2014, and Atezolizumab (Tecentriq^{®}), a PD-L1 immune checkpoint inhibitor developed by Genentech/Roche, was approved by the US FDA in 2016.

The PD-1 immune checkpoint inhibitor is a human anti-PD-1 monoclonal antibody or human anti-PD-L1 monoclonal antibody which blocks the lymphocyte negative regulation (interaction between PD-1 and PD-L1 and PD-L2 ligands) mediated by PD-1, and has an action mechanism of enhancing immunity to recognize cancer cells as a foreign material and remove the cancer cells.

Pembrolizumab has been FDA-approved for the treatment of malignant melanoma, non-small cell lung cancer, head and neck squamous cell cancer, typical Hodgkin's lymphoma, urothelial carcinoma, all solid cancers showing deficient mismatch repair (dMMR) or microsatellite instability-high (MSI-High) and gastric and gastroesophageal junction adenocarcinoma, and nivolumab has shown clinical benefits in clinical studies on various advanced cancers, and thus has recently been approved as a drug for melanoma, non-small cell lung cancer, kidney cancer, and the like, and has been widely used.

Furthermore, there is a "PD-L1 IHC 22C3 pharmDx test" that collects and analyzes a gastric and gastroesophageal junction adenocarcinoma tissue as a companion diagnosis test method to determine whether pembrolizumab efficiently exhibits anticancer effects as an immune anticancer drug in specific patients. The PD-L1 IHC 22C3 pharmDx test is one of the immunohistochemistry (IHC) methods, and is a test method used to screen a group of patients suitable for the treatment of pembrolizumab by observing the expression rate of PD-Li in biopsied cancer cells using a PD-L1 IHC 22C3 pharmDx product manufactured by the DAKO Corporation. Similarly, there are "VENTANA PD-L1 (SP263) Assay test", "PD-L1 IHC 28-8 pharmDx test", and the like as a companion diagnosis test method for nivolumab.

However, such existing companion diagnosis tests measure the expression of PD-L1 by removing some of the tissue (405 µm) of the tumor of a patient, but have problems in that the expression level of PD-L1 may vary depending on the tissue sampling of patients, and not only it takes 3 weeks or more to obtain an analysis result, but also the accuracy of screening a group of patients who show an effect for the immune anticancer drug somewhat deteriorates.

Accordingly, the present inventors developed a companion diagnosis biomarker composition and a companion diagnosis kit containing the same, the composition predicting a therapeutic response to at least one immune checkpoint inhibitor among a PD-1 immune checkpoint inhibitor and a PD-L1 immune checkpoint inhibitor not only through the measurement in tissues, but also through proteomic analysis of blood in order to overcome the technical limitations of such companion diagnosis tests.

### [Disclosure]

### [Technical Problem]

To solve the problems as described above, an object of the present invention is to provide a companion diagnosis biomarker composition for predicting a therapeutic response to at least one immune checkpoint inhibitor from among a Pd-1 immune checkpoint inhibitor and a PD-L1 immune checkpoint inhibitor not only through companion diagnosis through cancer patient tissues, but also through proteomic analysis of cancer patient blood.

In addition, a second object of the present invention is to provide a companion diagnosis biomarker composition and a companion diagnosis kit containing the same, the composition being for predicting a therapeutic response to at least one immune checkpoint inhibitor from among a PD-1 immune checkpoint inhibitor and a PD-L1 immune checkpoint inhibitor, which have a high accuracy of screening a group of responders who show an effect in anticancer treatment through at least one immune checkpoint inhibitor from among the PD-1 immune checkpoint inhibitor and the PD-L1 immune checkpoint inhibitor and a group of non-responders who do not show an effect in the anticancer treatment.

Furthermore, a third object of the present invention is to provide a companion diagnosis biomarker composition and a companion diagnosis kit containing the same, the composition being capable of more accurately determining a group of patients suitable for an anticancer treatment through at least one immune checkpoint inhibitor from among a PD-1 immune checkpoint inhibitor and a PD-L1 immune checkpoint inhibitor.

### [Technical Solution]

To achieve the objects as described above, the biomarker composition according to the present invention contains Complement Component C7 and is characterized by predicting the responsiveness to at least one immune checkpoint inhibitor from among a programmed cell death protein 1 (PD-1) immune checkpoint inhibitor and a programmed death-ligand 1 (PD-L1) immune checkpoint inhibitor for cancer cells.

Here, the companion diagnosis biomarker composition may further contain at least one biomarker selected from the group consisting of a histidine-rich glycoprotein (HRG), a zinc-alpha-2-glycoprotein (AZGP1), Complement Component C5, immunoglobulin kappa variable 4-1 (IHKV4-1), mannosyl-oligosaccharide 1,2-alpha-mannosidase 1A (MANIA), alpha-2-macroglobulin (A2M), osteopontin (SPP1) and hypoxia up-regulated protein 1 (HYOU1).

Here, the protein amount of Complement Component C7 may be measured by quantitative analysis.

Here, the protein amount of Complement Component C7 may be measured by any one method selected from the group consisting of protein mass analysis, protein chip analysis, an immune measurement method, a ligand binding assay, matrix desorption/ionization time of flight mass spectrometry (MALDI-TOF) analysis, surface enhanced laser desorption/ionization time of flight mass spectrometry (SELDI-TOF) analysis, a radioimmunoassay, a radial immunodiffusion method, an Ouchterlony immunodiffusion method, rocket immunoelectrophoresis, tissue immunostaining, a complement fixation assay method, 2-dimensional electrophoresis analysis, liquid chromatography-mass spectrometry (LC-MS), liquid chromatography-mass spectrometry/mass spectrometry (LC-MS/MS), western blot and enzyme linked immunosorbent assay (ELISA).

Here, for Complement Component C7, the amount of protein may be decreased in a group of responders who show an effect in anticancer treatment through the PD-1 immune checkpoint inhibitor compared to a group of non-responders who do not show an effect in the anticancer treatment through the PD-1 immune checkpoint inhibitor.

Here, for Complement Component C7, the amount of protein may be increased in a group of non-responders who do not show an effect in anticancer treatment through the PD-1 immune checkpoint inhibitor compared to a group of responders who show an effect in the anticancer treatment through the PD-1 immune checkpoint inhibitor.

Here, for Complement Component C7, the amount of protein may be decreased in a group of responders who show an effect in anticancer treatment through the PD-L1 immune checkpoint inhibitor compared to a group of non-responders who do not show an effect in the anticancer treatment through the PD-L1 immune checkpoint inhibitor.

Here, for Complement Component C7, the amount of protein may be increased in a group of non-responders who do not show an effect in anticancer treatment through the PD-L1 immune checkpoint inhibitor compared to a group of responders who show an effect in the anticancer treatment through the PD-L1 immune checkpoint inhibitor.

Here, a cutoff value for the protein amount of Complement Component C7 may be in a range of 100 to 110 µg/mL.

Here, when the protein amount of Complement Component C7 is equal to or more than the cutoff value, the responsiveness to the PD-1 immune checkpoint inhibitor for cancer cells may be low.

Here, when the protein amount of Complement Component C7 is less than the cutoff value, the responsiveness to the PD-1 immune checkpoint inhibitor for cancer cells may be high.

Here, when the protein amount of Complement Component C7 is equal to or more than the cutoff value, the responsiveness to the PD-L1 immune checkpoint inhibitor for cancer cells may be low.

Here, when the protein amount of Complement Component C7 is less than the cutoff value, the responsiveness to the PD-L1 immune checkpoint inhibitor for cancer cells may be high.

Here, the companion diagnosis biomarker composition may be extracted from any one selected from the group consisting of blood, serum, plasma and tissue.

Here, the companion diagnosis biomarker composition may be extracted from blood.

Here, the companion diagnosis biomarker composition may be extracted from tissue.

Here, the companion diagnosis biomarker composition may further contain a programmed death-ligand 1 (PD-L1) protein.

Here, the cancer cells may be cancer cells corresponding to carcinomas specifically responding to at least one immune checkpoint inhibitor from among a PD-1 immune checkpoint inhibitor and a PD-L1 immune checkpoint inhibitor.

Here, the cancer cells may be cancer cells corresponding to any one carcinoma selected from the group consisting of lung cancer, liver cancer, gastric cancer, gastric and gastroesophageal junction adenocarcinoma, skin melanoma, head and neck cancer, bone cancer, pancreatic cancer, skin cancer, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, chronic or acute leukemia, solid tumors of childhood, differentiated lymphoma, bladder cancer, kidney cancer, renal cell carcinoma, renal pelvic carcinoma, primary central nervous system lymphoma, spinal cord tumors, brainstem glioma and pituitary adenoma.

Here, the cancer cells may be cancer cells corresponding to lung cancer or liver cancer.

Here, the companion diagnosis biomarker composition may be administered simultaneously or sequentially in combination with at least one immune checkpoint inhibitor from among the PD-1 immune checkpoint inhibitor and the PD-L1 immune checkpoint inhibitor.

Meanwhile, the companion diagnosis kit according to the present invention may contain the companion diagnosis biomarker composition according to the present invention.

### [Advantageous Effects]

The companion diagnosis biomarker composition of the present invention as described above and the companion diagnosis kit containing the same have an effect of being able to predict a therapeutic response to at least one immune checkpoint inhibitor from among a PD-1 immune checkpoint inhibitor and a PD-L1 immune checkpoint inhibitor not only through a companion diagnosis through cancer patient tissues, but also through proteomic analysis of cancer patient blood.

In addition, the companion diagnosis biomarker composition according to the present invention and the companion diagnosis kit containing the same have an advantage of having high accuracy in screening a group of responders who show an effect in anticancer treatment through at least one immune checkpoint inhibitor from among a PD-1 immune checkpoint inhibitor and a PD-L1 immune checkpoint inhibitor and a group of non-responders who do not show an effect in the anticancer treatment.

Furthermore, the companion diagnosis biomarker composition according to the present invention and the companion diagnosis kit containing the same have an effect of being able to more accurately determine a group of patients suitable for an anticancer treatment through at least one immune checkpoint inhibitor from among a PD-1 immune checkpoint inhibitor and a PD-L1 immune checkpoint inhibitor.

### [Description of Drawings]

FIGS. 1A to 1I are graphs illustrating the companion diagnosis results of predicting the responsiveness to at least one immune checkpoint inhibitor from among a PD-1 immune checkpoint inhibitor and a PD-L1 immune checkpoint inhibitor for cancer cells for 9 companion diagnosis biomarkers of Complement Component C7, HRG AZGP1, Complement Component C5, IGKV4-1, MANIA, A2M, SPP1 and HYOU1, respectively.
FIG. 2A is a graph illustrating the companion diagnosis test results for 10 lung cancer patients through the companion diagnosis biomarker composition according to an exemplary embodiment of the present invention.
FIG. 2B is a graph illustrating the companion diagnosis test results for 11 lung cancer patients through the companion diagnosis biomarker composition according to an exemplary embodiment of the present invention.
FIG. 2C is a graph illustrating the companion diagnosis test results for a total of 21 lung cancer patients through the companion diagnosis biomarker composition according to an exemplary embodiment of the present invention.
FIG. 3 is a graph illustrating the companion diagnosis test results when the companion diagnosis biomarker composition according to an exemplary embodiment of the present invention further contains a biomarker IGKV4-1.

### [Modes of the Invention]

The companion diagnosis biomarker composition of the present invention contains Complement Component C7 and is characterized by predicting the responsiveness to at least one immune checkpoint inhibitor from among a PD-1 immune checkpoint inhibitor and a PD-L1 immune checkpoint inhibitor for cancer cells.

Terms used in the present application are used only to describe specific exemplary embodiments. For this reason, singular expressions include plural expressions unless the context clearly indicates otherwise. In addition, it should be noted that a term "include" or "comprise" used in the present application is used to indicate the presence of a feature, a step, a function, a component or a combination thereof described in the specification, but not used to preclude the presence of other features, steps, functions, components, or combinations thereof.

Meanwhile, unless defined otherwise, it should be considered that all terms used in the present specification have the same meaning as commonly understood by a person with ordinary skill in the art to which the present invention pertains. Thus, unless explicitly defined in the present specification, certain terms should not be construed in an overly ideal or formal sense.

As used herein, "companion diagnosis" refers to a diagnostic technique for predicting the responsiveness of a patient to a specific drug treatment in advance.

As used herein, "companion diagnosis biomarker" is an index for predicting the responsiveness of a patient to a specific drug treatment in advance, and may include proteins, DNA, RNA, metabolites and the like. That is, the companion diagnosis biomarker means a marker that can distinguish normal or pathological conditions, predict a therapeutic response, and objectively measure the therapeutic response in the case of a specific disease or cancer.

As used herein, "immune barrier inhibitor" is a synonym for an immune checkpoint inhibitor, and is a drug that attacks cancer cells by blocking the activation of an immune checkpoint protein involved in the suppression of T cells to activate T cells, at least one immune checkpoint inhibitor from among a PD-1 checkpoint inhibitor and a PD-L1 checkpoint inhibitor is disclosed in the present specification, and the PD-1 immune checkpoint inhibitor or PD-L1 immune checkpoint inhibitor may be pembrolizumab, nivolumab or atezolizumab, but is not limited thereto.

### <Companion diagnosis biomarker composition>

As a result of research to solve the above-described problems, the present inventors have devised the following invention. The present specification discloses a companion diagnosis biomarker composition which contains Complement Component C7 and is characterized by predicting the responsiveness to at least one immune checkpoint inhibitor from among a PD-1 immune checkpoint inhibitor and a PD-L1 immune checkpoint inhibitor for cancer cells.

The *in vivo* immune function regulates the overall T cell function by recognizing antigens and simultaneously regulating such co-stimulatory signals and co-inhibitory signals. Therefore, immune cells sense tumor-specific antigens expressed by changes such as mutations that occur in cancer cells and remove cancer cells. However, in order to avoid immune attack, cancer cells attempt to suppress the immune function by changing a tumor microenvironment and to achieve immune escape through T cell immune tolerance, immuno-editing or the like. As one of these escape strategies, the function of T cells is suppressed by changing the function of an immune checkpoint, that is, the immune checkpoint is a protein that interferes with the destruction of cancer cells, and cancer escapes the attack of T cells by activating an inhibitory immune checkpoint.

In such an immune response of T cells, the interaction between a co-stimulatory receptor that acts as an accelerator, a co-inhibitory receptor that acts as a brake, and a ligand that binds to each receptor is very elaborately operated in time and space. Meanwhile, the PD-1 of T cells regulates the function of T cells in peripheral tissues through PD-L1/PD-L2 of cancer cells. That is, when PD-L1 of cancer cells and PD-1 of T cells bind to each other, T cells lose their functions and die. Unlike existing immune therapeutic agents such as cytokine therapeutic agents and anticancer vaccines, the immune checkpoint inhibitor prevents the formation of immunological synapses by binding to the binding sites of cancer cells to block immune escape signals, and thus has a mechanism by which T cells which are not hindered by immune escape destroy cancer cells. That is, when a PD-1 antibody or PD-L1 antibody binds in advance between the binding sites of PD-L1 and PD-1, immune escape signals are blocked, and T cells that do not receive the immune escape signals kill cancer cells.

The companion diagnosis biomarker composition according to the present invention contains Complement Component C7, and the C7 may be used as a companion diagnosis biomarker of predicting the responsiveness to at least one immune checkpoint inhibitor from among a PD-1 checkpoint inhibitor and a PD-L1 checkpoint inhibitor for cancer cells.

Complement Component C7 included in the companion diagnosis biomarker composition according to the present invention serves to regulate the antigen-antibody immune response of the human body as a biomarker, and lyses pathogens by forming a membrane attack complex (MAC). Complement Component C7 is a protein involved in immune responses and lysis according to gene ontology classification, and the genetic information thereof can be found at GenBank (Accession No.), Uniprot, and the like. However, the direct correlation of Complement Component C7 in predicting the responsiveness to the PD-1 immune checkpoint inhibitor or PD-L1 immune checkpoint inhibitor for cancer cells has not been disclosed anywhere.

In addition, the companion diagnosis biomarker composition according to the present invention may further contain an agent which measures the protein amount of Complement Component C7.

Meanwhile, the companion diagnosis biomarker composition may further contain at least one biomarker selected from the group consisting of a histidine-rich glycoprotein (HRG), a zinc-alpha-2-glycoprotein (AZGP1), Complement Component C5, immunoglobulin kappa variable 4-1 (IHKV4-1), mannosyl-oligosaccharide 1,2-alpha-mannosidase 1A (MANIA), alpha-2-macroglobulin (A2M), osteopontin (SPP1) and hypoxia up-regulated protein 1 (HYOU1).

A histidine-rich glycoprotein (HRG), a zinc-alpha-2-glycoprotein (AZGP1), Complement Component C5, immunoglobulin kappa variable 4-1 (IHKV4-1), mannosyl-oligosaccharide 1,2-alpha-mannosidase 1A (MANIA), alpha-2-macroglobulin (A2M), osteopontin (SPP1) and hypoxia up-regulated protein 1 (HYOU1) contained in the companion diagnosis biomarker composition according to the present invention are proteins which serve to regulate the antigen-antibody immune response of the human body as biomarkers and are involved in immune responses and lysis according to gene ontology classification, and the genetic information thereof can be found at GenBank (Accession No.), Uniprot, and the like. However, the direct correlation of the HRG, AZGP1, Complement Component C5, IHKV4-1, MANIA, A2M and HYOU1 in predicting the responsiveness to the PD-1 immune checkpoint inhibitor or PD-L1 immune checkpoint inhibitor for cancer cells has not been disclosed anywhere.

Furthermore, the companion diagnosis biomarker composition according to the present invention may further contain an agent which measures the amount of protein of at least one biomarker selected from the group consisting of HRG, AZGP1, Complement Component C5, IHKV4-1, MANIA, A2M and HYOU1.

Meanwhile, the protein amount of Complement Component C7 in the companion diagnosis biomarker composition according to the present invention may be measured by quantitative analysis. Furthermore, for the protein amount of Complement Component C7, qualitative analysis, quantitative analysis or both the qualitative analysis and the quantitative analysis may be performed through a proteomic analysis method.

Similarly, the protein amount of at least one biomarker selected from the group consisting of HRG, AZGP1, Complement Component C5, IHKV4-1, MANIA, A2M and HYOU1 in the companion diagnosis biomarker composition according to the present invention may be measured by quantitative analysis. Furthermore, for the protein amount of at least one biomarker protein selected from the group consisting of HRG, AZGP1, Complement Component C5, IHKV4-1, MANIA, A2M and HYOU1, qualitative analysis, quantitative analysis or both the qualitative analysis and the quantitative analysis may also be performed through a proteomic analysis method.

As used herein, the "protein amount" refers to a process of confirming the presence or absence of and the degree of measurement of proteins in the blood of a cancer patient corresponding to a carcinoma specifically responding to at least one immune checkpoint inhibitor from among a PD-1 immune checkpoint inhibitor and a PD-L1 immune checkpoint inhibitor by treating the cancer patient with the at least one immune checkpoint inhibitor from among the PD-1 immune checkpoint inhibitor and the PD-L1 immune checkpoint inhibitor. It is possible not only to confirm the amount of protein using antibodies, interacting proteins, ligands, nanoparticles or aptamers that specifically bind to the protein or peptide fragment, but also to include all detection means having affinity specific to the corresponding protein or peptide fragment. More preferably, the protein amount itself can be measured without using antibodies, interacting proteins, ligands, nanoparticles or aptamers.

In the companion diagnosis biomarker composition according to the present invention, as a method of measuring the protein amount or a comparative analysis method of the protein amount, it is preferred to measure or comparatively analyze the protein amount by any one method selected from the group consisting of protein mass analysis, protein chip analysis, an immune measurement method, a ligand binding assay, matrix desorption/ionization time of flight mass spectrometry (MALDI-TOF) analysis, surface enhanced laser desorption/ionization time of flight mass spectrometry (SELDI-TOF) analysis, a radioimmunoassay, a radial immunodiffusion method, an Ouchterlony immunodiffusion method, rocket immunoelectrophoresis, tissue immunostaining, a complement fixation assay method, 2-dimensional electrophoresis analysis, liquid chromatography-mass spectrometry (LC-MS), liquid chromatography-mass spectrometry/mass spectrometry (LC-MS/MS), western blot and enzyme linked immunosorbent assay (ELISA), but the method is not limited thereto.

Meanwhile, in the companion diagnosis biomarker composition according to the present invention, the agent which measures the protein amount of Complement Component C7 may include at least one selected from the group consisting of antibodies, interacting proteins, ligands, nanoparticles and aptamers which specifically bind to the proteins of Complement Component C7. The antibody means a specific protein molecule directed against an antigenic site, but for the purpose of the present invention, the antibody refers to an antibody that specifically binds to the protein of Complement Component C7, and includes all of polyclonal antibodies, monoclonal antibodies and recombinant antibodies. The production of the antibody can be readily produced using a technique widely known in the art. Further, the antibody includes not only a complete form having two full-length light chains and two full-length heavy chains, but also the functional fragments of the antibody molecule. The functional fragments of the antibody molecule refer to the fragments having at least a function of binding antigens, and examples thereof include Fab, F(ab'), F(ab') 2, Fv, and the like.

In addition, in the companion diagnosis biomarker composition according to the present invention, the agent which measures the protein amount of at least one biomarker selected from the group consisting of HRG, AZGP1, Complement Component C5, IHKV4-1, MANIA, A2M and HYOU1 may include at least one selected from the group consisting of antibodies, interacting proteins, ligands, nanoparticles and aptamers which specifically bind to proteins of at least one biomarker selected from the group consisting of HRG, AZGP1, Complement Component C5, IHKV4-1, MANIA, A2M and HYOU1.

As used herein, the "aptamer" refers to a biopolymer material that suppresses the interaction of proteins through three-dimensional binding to a specific target protein in the form of single or double helix DNA or RNA, and has a characteristic of binding to various target molecules. Typically, the aptamers may be small nucleic acids with a length of 15 to 50 bases that are folded into defined secondary and tertiary structures, for example, stem-loop structures. It is preferred that the aptamers bind to a target high or low expression protein with a Kd of less than 10⁻⁶, 10⁻⁸, 10⁻¹⁰, or 10⁻¹². The aptamers can bind to high or low expression proteins with very high specificity, and the aptamers may consist of a plurality of ribonucleotide units and deoxyribonucleotide units, or a mixture of two types of nucleotide residues. Furthermore, the aptamers may additionally include one or more modified base, sugar or phosphate backbone units.

Meanwhile, Complement Component C7 included in the companion diagnosis biomarker composition according to the present invention is characterized in that the amount of protein is decreased in a group of responders who show an effect in anticancer treatment through the PD-1 immune checkpoint inhibitor compared to a group of non-responders who do not show an effect in the anticancer treatment through the PD-1 immune checkpoint inhibitor.

Conversely, Complement Component C7 included in the companion diagnosis biomarker composition according to the present invention is characterized in that the amount of protein is increased in a group of non-responders who do not show an effect in anticancer treatment through the PD-1 immune checkpoint inhibitor compared to a group of responders who show an effect in the anticancer treatment through the PD-1 immune checkpoint inhibitor.

Furthermore, Complement Component C7 included in the companion diagnosis biomarker composition according to the present invention is characterized in that the amount of protein is decreased in a group of responders who show an effect in anticancer treatment through the PD-L1 immune checkpoint inhibitor compared to a group of non-responders who do not show an effect in the anticancer treatment through the PD-L1 immune checkpoint inhibitor.

Conversely, Complement Component C7 included in the companion diagnosis biomarker composition according to the present invention is characterized in that the amount of protein is increased in a group of non-responders who do not show an effect in anticancer treatment through the PD-L1 immune checkpoint inhibitor compared to a group of responders who show an effect in the anticancer treatment through the PD-L1 immune checkpoint inhibitor.

In the companion diagnosis biomarker composition according to the present invention, a cutoff value for the protein amount of Complement Component C7 is preferably in a range of 90 to 120 µg/mL, and more preferably in a more specific range of 100 to 110 µg/mL, but is not limited thereto. The cutoff value may be arbitrarily set by a technician who performs a companion diagnosis test depending on the number of cancer patients to be measured. The cutoff value for the protein amount of Complement Component C7 means a value which becomes a criterion for determining the responsiveness to at least one immune checkpoint inhibitor from among a PD-1 immune checkpoint inhibitor and a PD-L1 immune checkpoint inhibitor for cancer cells, and more specifically a value which becomes a criterion for classifying patients into a group of responders who show an effect in the anticancer treatment through at least one immune checkpoint inhibitor from among a PD-1 immune checkpoint inhibitor and a PD-L1 immune checkpoint inhibitor and a group of non-responders who do not show an effect in the anticancer treatment.

Here, when the protein amount of Complement Component C7 is equal to or more than the cutoff value, the responsiveness to the PD-1 immune checkpoint inhibitor for cancer cells may be low. That is, as a result of performing a companion diagnosis which predicts the responsiveness to the PD-1 immune checkpoint inhibitor for cancer cells through the companion diagnosis biomarker composition according to the present invention, the case where the protein amount of Complement Component C7 is equal to or more than the cutoff value means that the responsiveness to the PD-1 immune checkpoint inhibitor for cancer cells is low. In this case, the patients can be classified into a group of non-responders who do not show an effect in the anticancer treatment through the PD-1 immune checkpoint inhibitor.

Conversely, when the protein amount of Complement Component C7 is less than the cutoff value, the responsiveness to the PD-1 immune checkpoint inhibitor for cancer cells may be high. That is, as a result of performing a companion diagnosis which predicts the responsiveness to the PD-1 immune checkpoint inhibitor for cancer cells through the companion diagnosis biomarker composition according to the present invention, the case where the protein amount of Complement Component C7 is less than the cutoff value means that the responsiveness to the PD-1 immune checkpoint inhibitor for cancer cells is high. In this case, the patients can be classified into a group of responders who show an effect in the anticancer treatment through the PD-1 immune checkpoint inhibitor.

When the protein amount of Complement Component C7 is equal to or more than the cutoff value, the responsiveness to the PD-L1 immune checkpoint inhibitor for cancer cells may be low. That is, as a result of performing a companion diagnosis which predicts the responsiveness to the PD-L1 immune checkpoint inhibitor for cancer cells through the companion diagnosis biomarker composition according to the present invention, the case where the protein amount of Complement Component C7 is equal to or more than the cutoff value means that the responsiveness to the PD-L1 immune checkpoint inhibitor for cancer cells is low. In this case, the patients can be classified into non-responders who do not show an effect in the anticancer treatment through the PD-L1 immune checkpoint inhibitor.

Conversely, when the protein amount of Complement Component C7 is less than the cutoff value, the responsiveness to the PD-L1 immune checkpoint inhibitor for cancer cells may be high. That is, as a result of performing a companion diagnosis which predicts the responsiveness to the PD-L1 immune checkpoint inhibitor for cancer cells through the companion diagnosis biomarker composition according to the present invention, the case where the protein amount of Complement Component C7 is less than the cutoff value means that the responsiveness to the PD-L1 immune checkpoint inhibitor for cancer cells is high. In this case, the patients can be classified into responders who show an effect in the anticancer treatment through the PD-L1 immune checkpoint inhibitor. In this regard, the specific content will be described in detail in the following {Examples and Evaluations}.

Meanwhile, at least one biomarker selected from the group consisting of HRG, AZGP1, Complement Component C5, IHKV4-1, MANIA, A2M and HYOU1 included in the companion diagnosis biomarker composition according to the present invention is characterized in that the amount of protein is decreased in a group of responders who show an effect in anticancer treatment through a PD-1 immune checkpoint inhibitor and/or a PD-L1 immune checkpoint inhibitor compared to a group of non-responders who do not show an effect in the anticancer treatment through the PD-1 immune checkpoint inhibitor and/or the PD-L1 immune checkpoint inhibitor.

Conversely, at least one biomarker selected from the group consisting of HRG, AZGP1, Complement Component C5, IHKV4-1, MANIA, A2M and HYOU1 included in the companion diagnosis biomarker composition according to the present invention is characterized in that the amount of protein is increased in a group of non-responders who do not show an effect in anticancer treatment through a PD-1 immune checkpoint inhibitor and/or a PD-L1 immune checkpoint inhibitor compared to a group of responders who show an effect in the anticancer treatment through the PD-1 immune checkpoint inhibitor and/or the PD-L1 immune checkpoint inhibitor.

Further, in the companion diagnosis biomarker composition according to the present invention, the cutoff value for the protein amount of at least one biomarker selected from the group consisting of HRG, AZGP1, Complement Complex C5, IHKV4-1, MANIA, A2M and HYOU1 is preferably in a range of 50 to 150 ug/mL, but is not limited thereto. The cutoff value for the protein amount of at least one biomarker selected from the group consisting of HRG, AZGP1, Complement Component C5, IHKV4-1, MANIA, A2M and HYOU1 means a value which becomes a criterion for determining the responsiveness to at least one immune checkpoint inhibitor from among a PD-1 immune checkpoint inhibitor and a PD-L1 immune checkpoint inhibitor for cancer cells, and more specifically a value which becomes a criterion for classifying patients into a group of responders who show an effect in the anticancer treatment through at least one immune checkpoint inhibitor from among a PD-1 immune checkpoint inhibitor and a PD-L1 immune checkpoint inhibitor and a group of non-responders who do not show an effect in the anticancer treatment.

Here, when the protein amount of at least one biomarker selected from the group consisting of HRG, AZGP1, Complement Component C5, IHKV4-1, MANIA, A2M and HYOU1 is equal to or more than the cutoff value, the responsiveness to the PD-1 immune checkpoint inhibitor and/or the PD-L1 immune checkpoint inhibitor for cancer cells may be low. That is, as a result of performing a companion diagnosis of predicting the responsiveness to the PD-1 immune checkpoint inhibitor and/or the PD-L1 immune checkpoint inhibitor for cancer cells through the companion diagnosis biomarker composition according to the present invention, the case where the protein amount of at least one biomarker selected from the group consisting of HRG, AZGP1, Complement Component C5, IHKV4-1, MANIA, A2M and HYOU1 is equal to or more than the cutoff value means that the responsiveness to the PD-1 immune checkpoint inhibitor and/or the PD-L1 immune checkpoint inhibitor for cancer cells is low. In this case, the patients can be classified into a group of non-responders who do not show an effect in the anticancer treatment through the PD-1 immune checkpoint inhibitor and/or the PD-L1 immune checkpoint inhibitor.

Conversely, when the protein amount of at least one biomarker selected from the group consisting of HRG, AZGP1, Complement Component C5, IHKV4-1, MANIA, A2M and HYOU1 is less than the cutoff value, the responsiveness to the PD-1 immune checkpoint inhibitor and/or the PD-L1 immune checkpoint inhibitor for cancer cells may be high. That is, as a result of performing a companion diagnosis of predicting the responsiveness to the PD-1 immune checkpoint inhibitor and/or the PD-L1 immune checkpoint inhibitor for cancer cells through the companion diagnosis biomarker composition according to the present invention, the case where the protein amount of at least one biomarker selected from the group consisting of HRG, AZGP1, Complement Component C5, IHKV4-1, MANIA, A2M and HYOU1 is less than the cutoff value means that the responsiveness to the PD-1 immune checkpoint inhibitor and/or the PD-L1 immune checkpoint inhibitor for cancer cells is high. In this case, the patients can be classified into a group of responders who show an effect in the anticancer treatment through the PD-1 immune checkpoint inhibitor and/or the PD-L1 immune checkpoint inhibitor.

Meanwhile, the companion diagnosis biomarker composition according to the present invention is characterized by being extracted from any one selected from the group consisting of blood, serum, plasma and tissue. In addition, as one effect of the present invention, it may be one feature of the present invention that the responsiveness to at least one immune checkpoint inhibitor from among a PD-1 immune checkpoint inhibitor and a PD-L1 immune checkpoint inhibitor can be predicted not only through a companion diagnosis by an existing tissue, but also through proteomic analysis of blood.

That is, it is preferred that the companion diagnosis biomarker composition according to the present invention is extracted from blood, but the present invention is not limited thereto. More specifically, the companion diagnosis biomarker composition according to the present invention has an effect that it is possible to predict a therapeutic response to at least one immune checkpoint inhibitor from among a PD-1 immune checkpoint inhibitor and a PD-L1 immune checkpoint inhibitor by performing a companion diagnosis through proteomic analysis of cancer patient blood.

Furthermore, the companion diagnosis biomarker composition according to the present invention may be extracted from tissue as described above, and in this case, the protein expression level of Complement Component C7 may be measured by quantitative analysis. Meanwhile, when the companion diagnosis biomarker composition according to the present invention is extracted from tissue, Complement Component C7 is characterized in that the expression level of the protein is decreased in a group of responders who show an effect in anticancer treatment through the PD-1 immune checkpoint inhibitor compared to a group of non-responders who do not show an effect in the anticancer treatment through the PD-1 immune checkpoint inhibitor.

Conversely, when the companion diagnosis biomarker composition according to the present invention is extracted from tissue, Complement Component C7 is characterized in that the expression level of the protein is increased in a group of non-responders who do not show an effect in anticancer treatment through the PD-1 immune checkpoint inhibitor compared to a group of responders who show an effect in the anticancer treatment through the PD-1 immune checkpoint inhibitor.

Furthermore, when the companion diagnosis biomarker composition according to the present invention is extracted from tissue, Complement Component C7 is characterized in that the expression level of the protein is decreased in a group of responders who show an effect in anticancer treatment through the PD-L1 immune checkpoint inhibitor compared to a group of non-responders who do not show an effect in the anticancer treatment through the PD-L1 immune checkpoint inhibitor.

Conversely, when the companion diagnosis biomarker composition according to the present invention is extracted from tissue, Complement Component C7 is characterized in that the expression level of the protein is increased in a group of non-responders who do not show an effect in anticancer treatment through the PD-L1 immune checkpoint inhibitor compared to a group of responders who show an effect in the anticancer treatment through the PD-L1 immune checkpoint inhibitor.

Meanwhile, when the companion diagnosis biomarker composition according to the present invention is extracted from tissue, at least one biomarker selected from the group consisting of HRG, AZGP1, Complement Component C5, IHKV4-1, MANIA, A2M and HYOU1 is characterized in that the expression level of the protein is decreased in a group of responders who show an effect in anticancer treatment through a PD-1 immune checkpoint compared to a group of non-responders who do not show an effect in the anticancer treatment through the PD-1 immune checkpoint inhibitor.

Conversely, when the companion diagnosis biomarker composition according to the present invention is extracted from tissue, at least one biomarker selected from the group consisting of HRG, AZGP1, Complement Component C5, IHKV4-1, MANIA, A2M and HYOU1 is characterized in that the expression level of the protein is increased in a group of non-responders who do not show an effect in anticancer treatment through a PD-1 immune checkpoint inhibitor compared to a group of responders who show an effect in the anticancer treatment through the PD-1 immune checkpoint inhibitor.

Further, when the companion diagnosis biomarker composition according to the present invention is extracted from tissue, at least one biomarker selected from the group consisting of HRG, AZGP1, Complement Component C5, IHKV4-1, MANIA, A2M and HYOU1 is characterized in that the expression level of the protein is decreased in a group of responders who show an effect in anticancer treatment through a PD-L1 immune checkpoint inhibitor compared to a group of non-responders who do not show an effect in the anticancer treatment through the PD-L1 immune checkpoint inhibitor.

Conversely, when the companion diagnosis biomarker composition according to the present invention is extracted from tissue, at least one biomarker selected from the group consisting of HRG, AZGP1, Complement Component C5, IHKV4-1, MANIA, A2M and HYOU1 is characterized in that the expression level of the protein is increased in a group of non-responders who do not show an effect in anticancer treatment through a PD-L1 immune checkpoint inhibitor compared to a group of responders who show an effect in the anticancer treatment through the PD-L1 immune checkpoint inhibitor.

In addition, when the companion diagnosis biomarker composition according to the present invention is extracted from tissue, the companion diagnosis biomarker composition may further contain a programmed death-ligand 1 (PD-L1) protein. Accordingly, it is possible to predict the therapeutic response of a cancer patient to at least one immune checkpoint inhibitor from among a PD-1 immune checkpoint inhibitor and a PD-L1 immune checkpoint inhibitor through the companion diagnosis biomarker composition according to the present invention along with the measurement of the expression level of the PD-L1 protein.

Meanwhile, in the companion diagnosis biomarker composition according to the present invention, the cancer cells are characterized by being cancer cells corresponding to carcinomas specifically responding to at least one immune checkpoint inhibitor from among a PD-1 immune checkpoint inhibitor and a PD-L1 immune checkpoint inhibitor. That is, according to the present invention, a companion diagnosis may be performed regardless of the type of carcinoma as long as anticancer treatment can be performed on the carcinoma through at least one immune checkpoint inhibitor from among a PD-1 immune checkpoint inhibitor and a PD-L1 immune checkpoint inhibitor.

In addition, in the companion diagnosis biomarker composition according to the present invention, the cancer cells are characterized by being cancer cells corresponding to any one carcinoma selected from the group consisting of lung cancer, liver cancer, gastric cancer, gastric and gastroesophageal junction adenocarcinoma, skin melanoma, head and neck cancer, bone cancer, pancreatic cancer, skin cancer, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, chronic or acute leukemia, solid tumors of childhood, differentiated lymphoma, bladder cancer, kidney cancer, renal cell carcinoma, renal pelvic carcinoma, primary central nervous system lymphoma, spinal cord tumors, brainstem glioma and pituitary adenoma.

More specifically, in the companion diagnosis biomarker composition according to the present invention, the cancer cells are preferably cancer cells corresponding to lung cancer or liver cancer, but are not limited thereto.

Meanwhile, the companion diagnosis biomarker composition according to the present invention is characterized by being administered simultaneously or sequentially in combination with at least one immune checkpoint inhibitor from a PD-1 immune checkpoint inhibitor and a PD-L1 immune checkpoint inhibitor.

### <Companion diagnosis kit>

Meanwhile, the present specification additionally discloses a companion diagnosis kit containing a companion diagnosis biomarker composition which contains Complement Component C7 and is characterized by predicting the responsiveness to at least one immune checkpoint inhibitor from among a PD-1 immune checkpoint inhibitor and a PD-L1 immune checkpoint inhibitor for cancer cells. The above-described <companion diagnosis biomarker composition> shall be applied mutatis mutandis to the details on the companion diagnosis biomarker composition in the companion diagnosis kit according to the present invention.

The companion diagnosis kit according to the present invention may contain the companion diagnosis biomarker composition according to the present invention.

The companion diagnosis kit according to the present invention is preferably any one kit selected from the group consisting of an enzyme linked immunosorbent assay (ELISA) kit, a protein chip kit, a rapid kit and a multiple reaction monitoring (MRM) kit, but is not limited thereto. In this case, any one kit selected from the group consisting of an ELISA kit, a protein chip kit, a rapid kit and a multiple reaction monitoring (MRM) kit may be a kit performed by known methods.

Furthermore, the companion diagnosis kit according to the present invention may further include one or more other constituent component compositions, solutions or devices suitable for the analytical method.

The companion diagnosis kit according to the present invention may be a diagnosis kit characterized by containing essential elements necessary for performing ELISA. The ELISA kit may include antibodies, interacting proteins, ligands, nanoparticles or aptamers that specifically bind to the protein or peptide fragment. The antibody is an antibody having high specificity and affinity for a protein of each complement component, and may be a monoclonal antibody, a polyclonal antibody or a recombinant antibody. Further, the ELISA kit may include antibodies, interacting proteins, ligands, nanoparticles or aptamers that specifically bind to the protein or peptide fragment, or antibodies specific for a control protein. In addition, the ELISA kit may include a reagent capable of detecting a bound antibody, for example, a labeled secondary antibody, chromophores, an enzyme (for example: an enzyme conjugated to the antibody) and a substrate thereof, or other materials capable of binding to the antibody, and the like.

Hereinafter, the details claimed in the present specification will be described in more detail with reference to the accompanying drawings and examples. However, since the drawings or examples presented in the present specification can be modified in various ways by those skilled in the art and have various forms, it should be noted that the description of the present specification does not limit the present invention to a specific disclosed form, but includes all equivalents or alternatives included in the spirit and technical scope of the present invention. Furthermore, the accompanying drawings are presented to allow those skilled in the art to more accurately understand the present invention, and may be shown in a form of exaggerated or reduced in size from the actual size.

### {Examples and Evaluations}

### Example 1. Selection of nine companion diagnosis biomarkers containing Complement Component C7

By data mining, nine biomarker candidates contained in the companion diagnosis biomarker composition according to the present invention were selected, and efficacy verification (validation stage) was performed on the selected nine biomarker candidates.

Ten blood samples (hereinafter, referred to as "Training Set"), in which the responsiveness of a lung cancer patient to a PD-1 immune checkpoint inhibitor or a PD-L1 immune checkpoint inhibitor against cancer cells had been already determined, were prepared. Here, the ten blood samples were eight blood samples of a group of responders who showed an effect in anticancer treatment through a PD-1 immune checkpoint inhibitor or a PD-L1 immune checkpoint inhibitor and two blood samples of a group of non-responders (progressive disease, PD) who did not show an effect in the anticancer treatment through the PD-1 immune checkpoint inhibitor or the PD-L1 immune checkpoint inhibitor. Further, the eight blood samples from the group of responders who showed an effect in the anticancer treatment through the PD-1 immune checkpoint inhibitor or the PD-L1 immune checkpoint inhibitor were classified into five blood samples of a group of responders (partial response, PR) who showed a high effect in the anticancer treatment through the PD-1 immune checkpoint inhibitor or the PD-L1 immune checkpoint inhibitor and three blood samples of a group of responders (stable disease, SD) who showed a low effect in the anticancer treatment through the PD-1 immune checkpoint inhibitor or the PD-L1 immune checkpoint inhibitor.

Specific information on the ten blood samples, in which the responsiveness of a lung cancer patient to a PD-1 immune checkpoint inhibitor or a PD-L1 immune checkpoint inhibitor against cancer cells had been already determined is shown in the following Table 1. However, in the following Table 1, PD means a group of non-responders who do not show an effect in the anticancer treatment through the PD-1 immune checkpoint inhibitor or the PD-L1 immune checkpoint inhibitor, PR means a group of responders who show a high effect in the anticancer treatment through the PD-1 immune checkpoint inhibitor or the PD-L1 immune checkpoint inhibitor, and SD means a group of responders who show a low effect in the anticancer treatment through the PD-1 immune checkpoint inhibitor or the PD-L1 immune checkpoint inhibitor. In addition, in the following Table 1, M represents male and F represents female.

**[Table 1]**

| No. | Group | Gender | Age | Immune checkpoint inhibitor |
|---|---|---|---|---|
| 1 | PR | M | 52 | Nivolumab, PD-1 Checkpoint Inhibitor |
| 2 | | F | 69 | Pembrolizumab, PD-1 Checkpoint Inhibitor |
| 3 | | M | 62 | Atezolizumab, PD-L1 Checkpoint Inhibitor |
| 4 | | M | 58 | Nivolumab, PD-1 Checkpoint Inhibitor |
| 5 | | M | 65 | Nivolumab, PD-1 Checkpoint Inhibitor |
| 6 | SD | M | 60 | Pembrolizumab, PD-1 Checkpoint Inhibitor |
| 7 | | F | 58 | Pembrolizumab, PD-1 Checkpoint Inhibitor |
| 8 | | F | 71 | Pembrolizumab, PD-1 Checkpoint Inhibitor |
| 9 | PD | M | 69 | Nivolumab, PD-1 Checkpoint Inhibitor |
| 10 | | M | 70 | Atezolizumab, PD-L1 Checkpoint Inhibitor |

In order to predict the responsiveness to a PD-1 immune checkpoint inhibitor or a PD-L1 immune checkpoint inhibitor in the ten blood samples, in which the responsiveness to the PD-1 immune checkpoint inhibitor or the PD-L1 immune checkpoint inhibitor for cancer cells of a lung cancer patient had been already determined, after serum was extracted from the ten blood samples, the protein amounts of the nine selected biomarker candidates were measured, respectively, by subjecting the extracted serum to a pre-treatment process and performing proteomic analysis on the ten samples of serum using TMT10 isobaric compounds. However, here, in the protein analysis, the protein amounts for the nine biomarker candidates were measured by quantitative analysis.

In this case, each cutoff value of protein amounts measured for the nine biomarker candidates was set to 100 µg/mL and when the protein amount of each of the nine biomarker candidates was equal to or more than a cutoff value, samples were classified into a group of non-responders (progressive disease, PD) who did not show an effect in the anticancer treatment through the PD-1 immune checkpoint inhibitor or the PD-L1 immune checkpoint inhibitor, and when the protein amount was less than the cutoff value, samples were classified into a group of responders (partial response, PR) who showed a high effect in the anticancer treatment through the PD-1 immune checkpoint inhibitor or the PD-L1 immune checkpoint inhibitor and a group of responders (stable disease, SD) who showed a low effect in the anticancer treatment through the PD-1 immune checkpoint inhibitor or the PD-L1 immune checkpoint inhibitor according to the protein measurement amounts of the nine biomarker candidates (hereinafter, referred to as 'Example 1').

### Evaluation 1. Evaluation of efficacy of companion diagnosis biomarker composition containing Complement Component C7

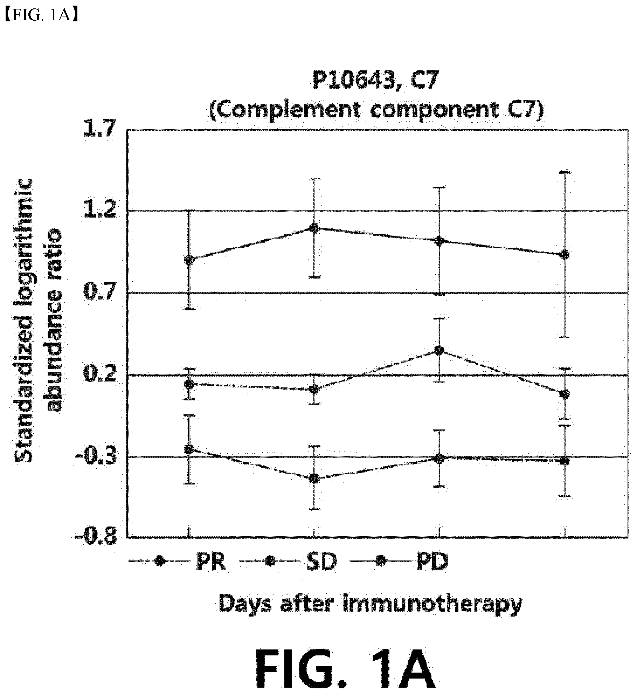

FIGS. 1A to 1I are graphs illustrating the companion diagnosis results of predicting the reactivity of at least one immune checkpoint inhibitor from among a PD-1 immune checkpoint inhibitor and a PD-L1 immune checkpoint inhibitor to cancer cells for 9 companion diagnosis biomarkers of Complement Component C7, HRG, AZGP1, Complement Component C5, IGKV4-1, MANIA, A2M, SPP1 and HYOU1, respectively.

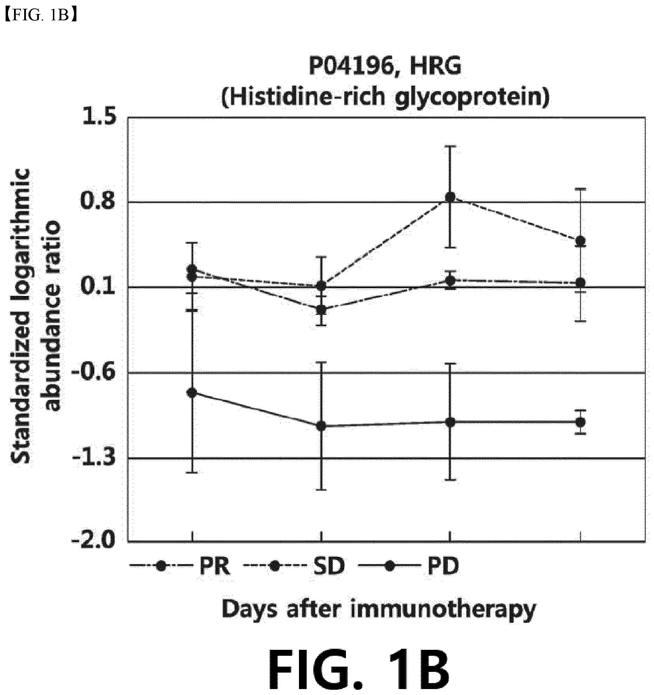

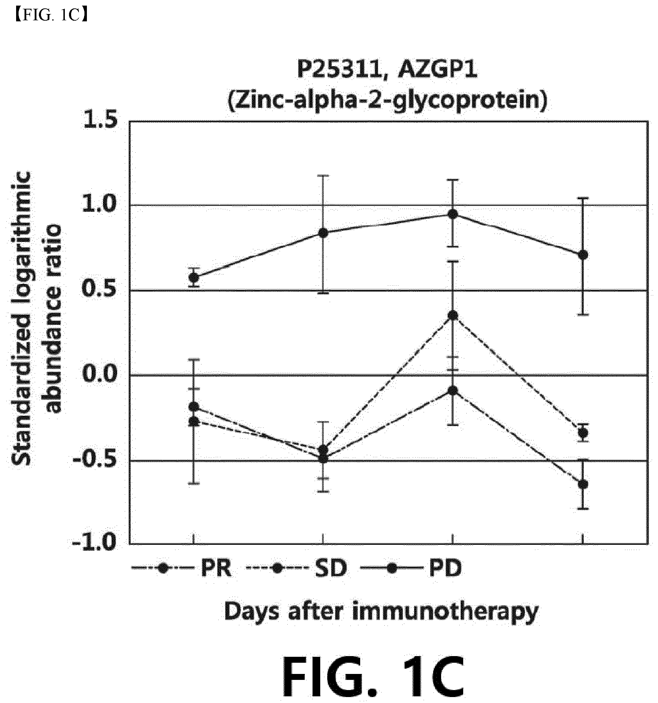

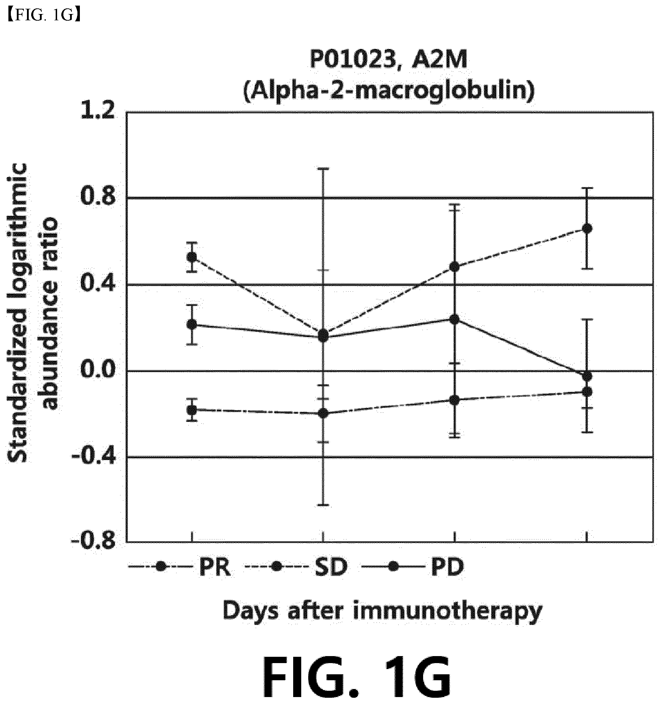

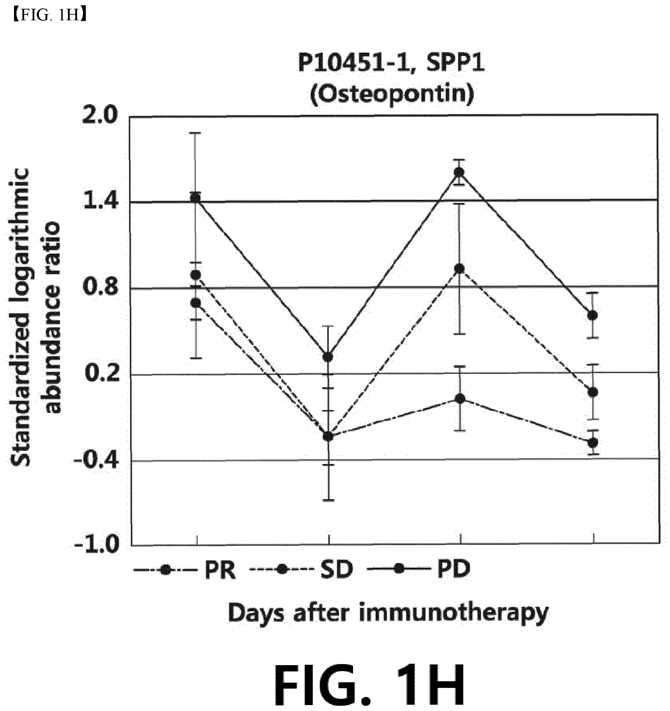

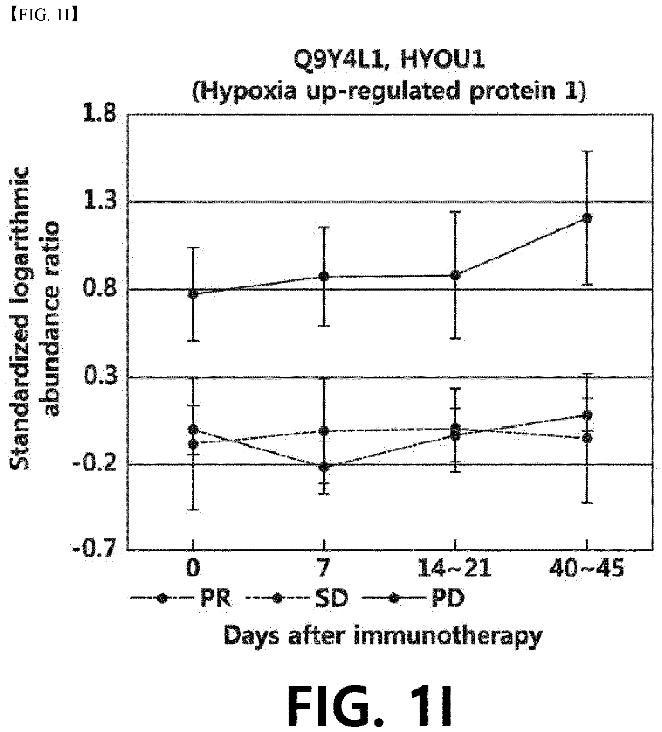

More specifically, FIG. 1A, FIG. 1B, FIG. 1C, FIG. ID, FIG. IE, FIG. IF, FIG. 1G, FIG. 1H, and FIG. 1I are graphs illustrating the companion diagnosis results of predicting the responsiveness to the PD-1 immune checkpoint inhibitor for cancer cells for biomarkers C7, HRG, AZGP1, C5, IGKV4-1, MANIA, A2M, SPP1, and HYOU1, respectively.

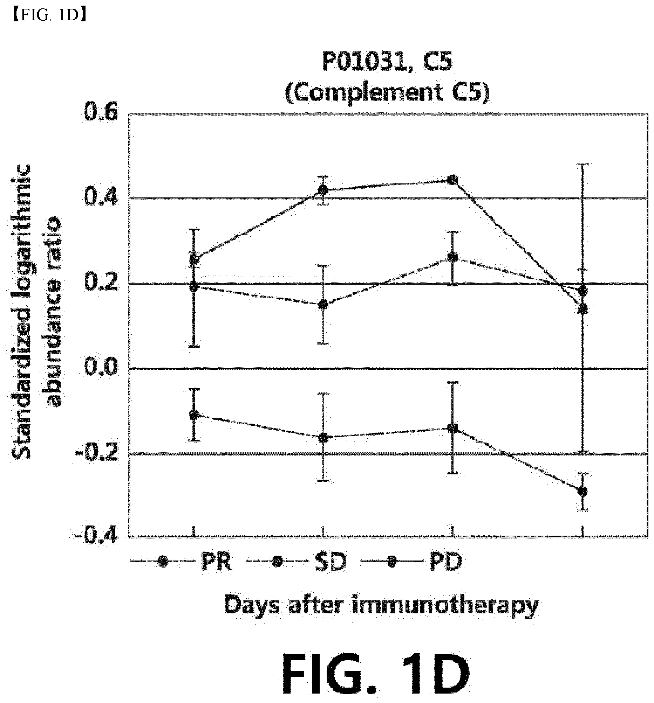

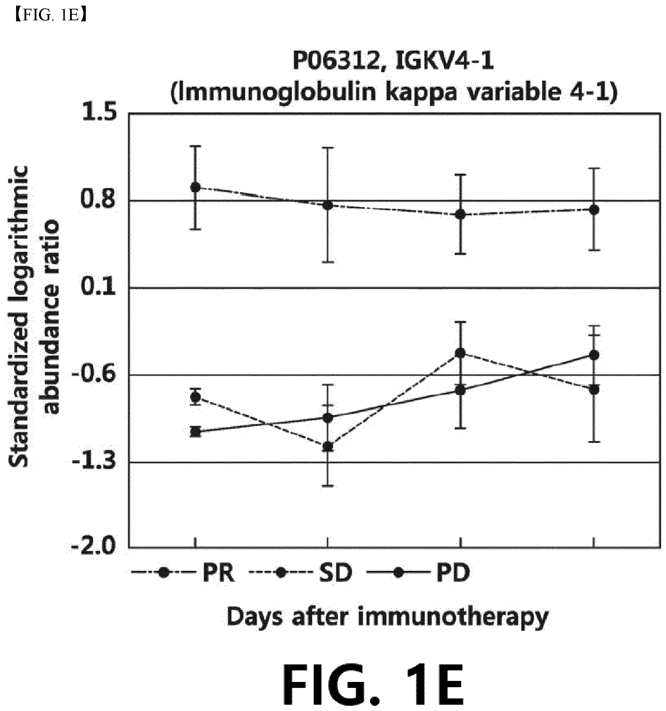

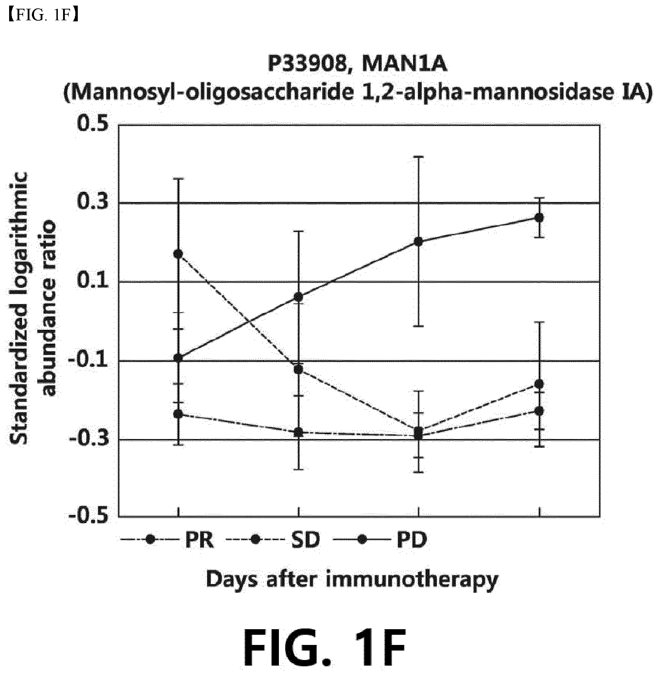

Referring to FIG. 1, as a result of protein analysis on ten blood samples, it can be confirmed that even after several days, the protein measurement amounts of biomarkers were constant, and the companion diagnosis results of biomarker C7 showing a constant tendency when the protein amount of the biomarker was equal to or more than the cutoff value and when the protein amount of the biomarker was less than the cut off value were the best. Accordingly, the biomarker C7 was specified as the companion diagnosis biomarker composition according to the present invention, and it can be presumed that when the other biomarkers HRG, AZGP1, C5, IHKV4-1, MANIA, A2M and HYOU1 are used in combination with C7, excellent companion diagnosis results can be exhibited as the biomarker compositions according to the present invention.

### Example 2. Companion diagnosis test through companion diagnosis biomarker composition according to present invention

Eleven blood samples (hereinafter, referred to as "Set") of lung cancer patients were prepared according to the results of Example 1, serum was extracted from each of the eleven blood samples, and then subjected to a pre-treatment process, and each of the protein amounts for Complement Component C7 was measured by performing proteomic analysis on the eleven samples of serum using TMT10 isobaric compounds. However, here, in the protein analysis, each of the protein amounts for Complement Component C7 was measured by quantitative analysis.

Specific information on the eleven blood samples from lung cancer patients is shown in the following Table 2. However, in the following Table 2, PD means a group of non-responders who do not show an effect in the anticancer treatment through the PD-1 immune checkpoint inhibitor or the PD-L1 immune checkpoint inhibitor, PR means a group of responders who show a high effect in the anticancer treatment through the PD-1 immune checkpoint inhibitor or the PD-L1 immune checkpoint inhibitor, and SD means a group of responders who show a low effect in the anticancer treatment through the PD-1 immune checkpoint inhibitor or the PD-L1 immune checkpoint inhibitor. In addition, in the following Table 2, M represents male and F represents female.

**[Table 2]**

| No. | Group | Gender | Age | Immune checkpoint inhibitor |
|---|---|---|---|---|
| 11 | PR | M | 59 | Pembrolizumab, PD-1 Checkpoint Inhibitor |
| 12 | | F | 85 | Pembrolizumab, PD-1 Checkpoint Inhibitor |
| 13 | SD | M | 65 | Pembrolizumab, PD-1 Checkpoint Inhibitor |
| 14 | | M | 47 | Nivolumab, PD-1 Checkpoint Inhibitor |
| 15 | | M | 71 | Nivolumab, PD-1 Checkpoint Inhibitor |
| 16 | PD | F | 61 | Pembrolizumab, PD-1 Checkpoint Inhibitor |
| 17 | | M | 66 | Pembrolizumab, PD-1 Checkpoint Inhibitor |
| 18 | | M | 62 | Nivolumab, PD-1 Checkpoint Inhibitor |
| 19 | | M | 45 | Nivolumab, PD-1 Checkpoint Inhibitor |
| 20 | | M | 75 | Atezolizumab, PD-L1 Checkpoint Inhibitor |
| 21 | | M | 55 | Pembrolizumab, PD-1 Checkpoint Inhibitor |

In this case, the cutoff value of the protein amount measured for Complement Component C7 was set to 100 µg/mL and when the protein amount of Complement Component C7 was equal to or more than a cutoff value, samples were classified into a group of non-responders (progressive disease, PD) who did not show an effect in the anticancer treatment through the PD-1 immune checkpoint inhibitor or the PD-L1 immune checkpoint inhibitor, and when the protein amount of Complement Component C7 was less than the cutoff value, samples were classified into a group of responders (partial response, PR) who showed a high effect in the anticancer treatment through the PD-1 immune checkpoint inhibitor or the PD-L1 immune checkpoint inhibitor and a group of responders (stable disease, SD) who showed a low effect in the anticancer treatment through the PD-1 immune checkpoint inhibitor or the PD-L1 immune checkpoint inhibitor according to the protein measurement amounts of Complement Component 7 (hereinafter, referred to as 'Example 2').

### Evaluation 2. Analysis and evaluation of companion diagnosis test results through companion diagnosis biomarker composition according to present invention

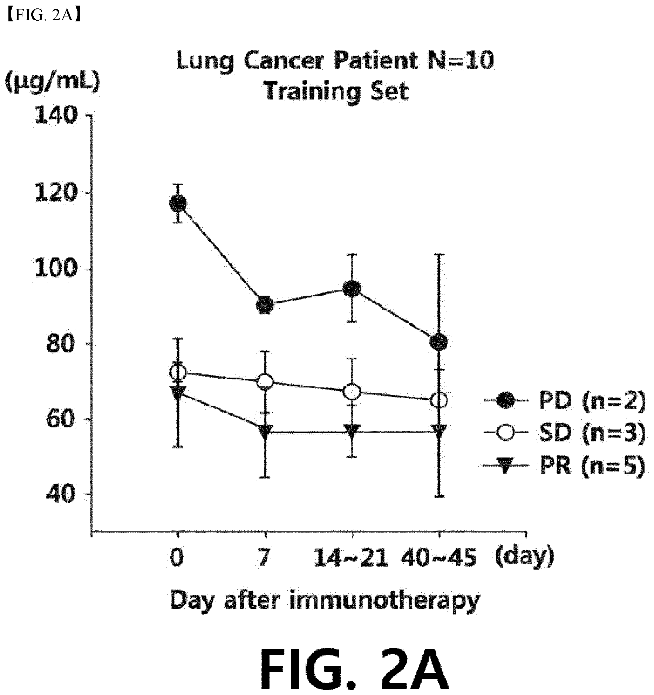

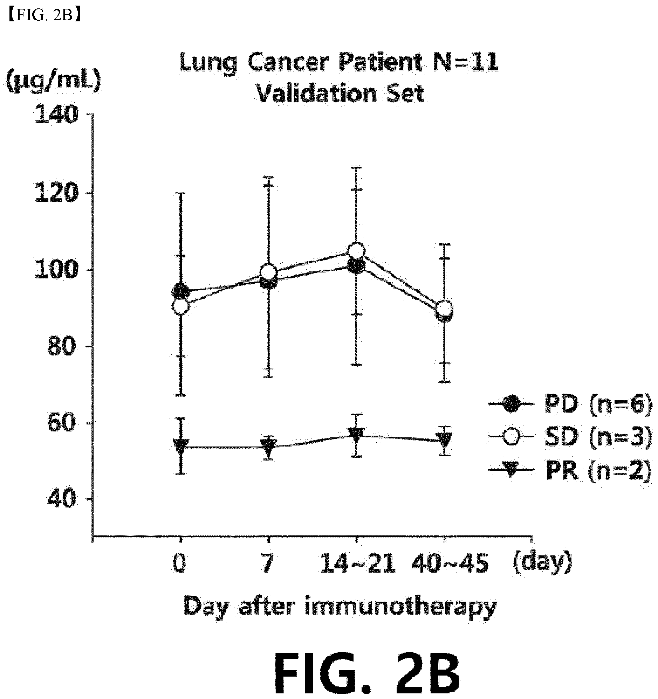

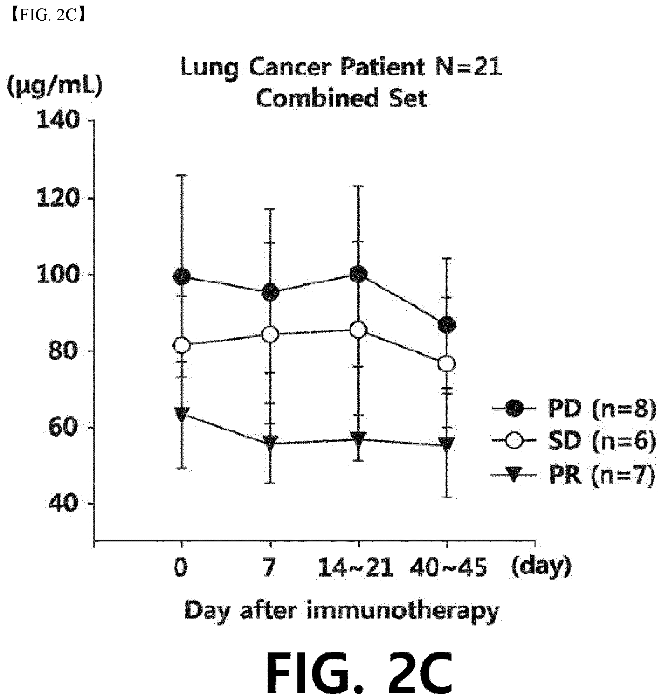

FIG. 2A is a graph illustrating the companion diagnosis test results for 10 lung cancer patients through the companion diagnosis biomarker composition according to an exemplary embodiment of the present invention. FIG. 2B is a graph illustrating the companion diagnosis test results for 11 lung cancer patients through the companion diagnosis biomarker composition according to an exemplary embodiment of the present invention. FIG. 2C is a graph illustrating the companion diagnosis test results for a total of 21 lung cancer patients through the companion diagnosis biomarker composition according to an exemplary embodiment of the present invention by.

More specifically, FIG. 2A illustrates the companion diagnosis test results through biomarker C7 in the training set of Example 1, FIG. 2B illustrates the companion diagnosis test results through Complement Component C7 in the validation set of Example 2, and FIG 2C illustrates the final result of combining the companion diagnosis test results through biomarker C7 in the training set of Example 1 and the companion diagnosis test results through Complement Component C7 in the validation set of Example 2.

Referring to FIG. 2, as a result of also performing a companion diagnosis test on the eleven blood samples of lung cancer patients of Example 2 through the companion diagnosis biomarker composition according to the present invention, it can be confirmed that the samples can be accurately classified into a group of non-responders (progressive disease, PD) who do not show an effect in the anticancer treatment through the PD-1 immune checkpoint inhibitor or the PD-L1 immune checkpoint inhibitor, a group of responders (partial response, PR) who show a high effect in the anticancer treatment through the PD-1 immune checkpoint inhibitor or the PD-L1 immune checkpoint inhibitor, and a group of responders (stable disease, SD) who show a low effect in the anticancer treatment through the PD-1 immune checkpoint inhibitor or the PD-L1 immune checkpoint inhibitor on the basis of a cutoff value according to the protein amount of Complement Component C7.

That is, it can be said that the companion diagnosis biomarker composition according to the present invention has an excellent effect in companion diagnosis which predicts the responsiveness to the PD-1 immune checkpoint inhibitor or the PD-L1 immune checkpoint inhibitor for cancer cells by containing Complement Component C7.

### Example 3. Companion diagnosis test through companion diagnosis biomarker composition according to present invention further containing biomarker IGKV4-1

Ten blood samples (hereinafter, referred to as "Training Set"), in which the responsiveness of a lung cancer patient to a PD-1 immune checkpoint inhibitor or a PD-L1 immune checkpoint inhibitor against cancer cells had been already determined, in Example 1 were prepared. Information on the ten blood samples is as described above in Example 1. In order to predict the responsiveness to a PD-1 immune checkpoint inhibitor or a PD-L1 immune checkpoint inhibitor in the ten blood samples, in which the responsiveness to the PD-1 immune checkpoint inhibitor or the PD-L1 immune checkpoint inhibitor for cancer cells of a lung cancer patient had been already determined, after serum was extracted from the ten blood samples, the protein amounts of Complement Component C7 and immunoglobulin kappa variable 4-1 (IGKV4-1) were measured, respectively by subjecting the extracted serum to a pre-treatment process and performing proteomic analysis on the ten samples of serum using TMT10 isobaric compounds. However, here, in the protein analysis, each of the protein amounts for biomarkers Complement Component C7 and IGKV4-1 was measured by quantitative analysis.

In this case, a relative ratio was obtained by dividing the protein amount of Complement Component C7 measured from each of the ten serum samples by the measured protein amount of IGKV4-1. The blood samples were classified into a group of non-responders (progressive disease, PD) who did not show an effect in the anticancer treatment through the PD-1 immune checkpoint inhibitor or the PD-L1 immune checkpoint inhibitor, a group of responders (partial response, PR) who showed a high effect in the anticancer treatment through the PD-1 immune checkpoint inhibitor or the PD-L1 immune checkpoint inhibitor, and a group of responders (stable disease, SD) who showed a low effect in the anticancer treatment through the PD-1 immune checkpoint inhibitor or the PD-L1 immune checkpoint inhibitor according to the relative ratio (hereinafter, referred to as 'Example 3').

### Evaluation 3. Analysis and evaluation of companion diagnosis test through companion diagnosis biomarker composition according to present invention further containing biomarker IGKV4-1

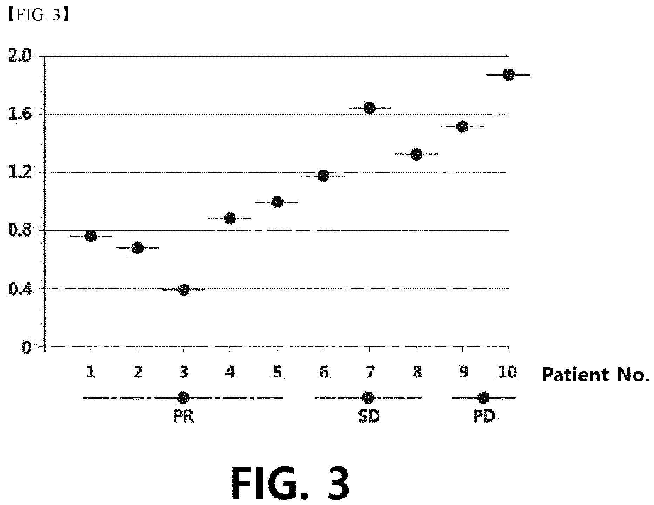

FIG. 3 is a graph illustrating the companion diagnosis test results when the companion diagnosis biomarker composition according to an exemplary embodiment of the present invention further contains a biomarker IGKV4-1.

Referring to FIG. 3, as a result of also performing a companion diagnosis test on the ten blood samples of lung cancer patients of Example 3 through the companion diagnosis biomarker composition according to the present invention, it can be confirmed that the samples can be accurately classified into a group of non-responders (progressive disease, PD) who do not show an effect in the anticancer treatment through the PD-1 immune checkpoint inhibitor or the PD-L1 immune checkpoint inhibitor, a group of responders (partial response, PR) who show a high effect in the anticancer treatment through the PD-1 immune checkpoint inhibitor or the PD-L1 immune checkpoint inhibitor, and a group of responders (stable disease, SD) who show a low effect in the anticancer treatment through the PD-1 immune checkpoint inhibitor or the PD-L1 immune checkpoint inhibitor by a predetermined reference value according to the relative ratio obtained by dividing the protein amount of Complement Component C7 by the protein amount of IGKV4-1. That is, the results according to Example 3 coincide with the information on the ten blood samples shown in Table 1 of Example 1.

That is, it can be confirmed that the companion diagnosis biomarker composition according to the present invention has not only an excellent effect in companion diagnosis which predicts the responsiveness to the PD-1 immune checkpoint inhibitor or the PD-L1 immune checkpoint inhibitor for cancer cells by containing Complement Component C7, but also a better effect in companion diagnosis which predicts the responsiveness to the PD-1 immune checkpoint inhibitor or the PD-L1 immune checkpoint inhibitor for cancer cells even when the companion diagnosis biomarker composition according to the present invention further contains a biomarker IGKV4-1.

It can be presumed that the same excellent companion diagnosis results as in Example 3 will be exhibited not only when Complement Component C7 further contains a biomarker IGKV4-1, but also when Complement Component C7 further contains at least one biomarker selected from the group consisting of a histidine-rich glycoprotein (HRG), a zinc-alpha-2-glycoprotein (AZGP1), Complement Component C5, mannosyl-oligosaccharide 1,2-alpha-mannosidase 1A (MANIA), alpha-2-macroglobulin (A2M), osteopontin (SPP1) and hypoxia up-regulated protein 1 (HYOU1). This can be inferred from the common point that the HRG, AZGP1, Complement Component C5, IHKV4-1, MANIA, A2M and HYOU1 serve to regulate the antigen-antibody immune response of the human body as biomarkers, and are proteins involved in immune responses and lysis according to gene ontology classification.

### Experimental Example 1. When cutoff value for protein measurement amount of Complement Component C7 in companion diagnosis biomarker composition according to present invention is 100 µg/mL (comparison with PD-L1 pharmDx test)

Twenty seven blood samples of lung cancer patients were prepared, serum was extracted from each of the twenty seven blood samples, and then subjected to a pre-treatment process, and each of the protein amounts for Complement Component C7 was measured by performing proteomic analysis on the twenty seven samples of serum using TMT10 isobaric compounds. However, here, in the protein analysis, each of the protein amounts for Complement Component C7 was measured by quantitative analysis.

In this case, the cutoff value of the protein amount measured for Complement Component C7 was set to 100 µg/mL and when the protein amount of Complement Component C7 was equal to or more than a cutoff value, samples were classified into a group of non-responders (progressive disease, PD) who did not show an effect in the anticancer treatment through at least one immune checkpoint inhibitor from among the PD-1 immune checkpoint inhibitor and the PD-L1 immune checkpoint inhibitor, and when the protein amount of Complement Component C7 was less than the cutoff value, samples were classified into a group of responders (partial response, PR) who showed a high effect in the anticancer treatment through at least one immune checkpoint inhibitor from among the PD-1 immune checkpoint inhibitor and the PD-L1 immune checkpoint inhibitor and a group of responders (stable disease, SD) who showed a low effect in the anticancer treatment through at least one immune checkpoint inhibitor from among the PD-1 immune checkpoint inhibitor and the PD-L1 immune checkpoint inhibitor according to the protein measurement amounts of Complement Component 7 (hereinafter, referred to as 'Experimental Example 1').

### Experimental Example 2. When cutoff value for protein measurement amount of Complement Component C7 in companion diagnosis biomarker composition according to present invention is 105 µg/mL (comparison with PD-L1 pharmDx test)

In the same manner as in Experimental Example 1 except that a cutoff value of the protein amount measured for Complement Component C7 was set to 105 µg/mL, when the protein amount of Complement Component C7 was equal to or more than the cutoff value, samples were classified into a group of non-responders (progressive disease, PD) who did not show an effect in the anticancer treatment through at least one immune checkpoint inhibitor from among the PD-1 immune checkpoint inhibitor and the PD-L1 immune checkpoint inhibitor, and when the protein amount of Complement Component C7 was less than the cutoff value, samples were classified into a group of responders (partial response, PR) who showed a high effect in the anticancer treatment through at least one immune checkpoint inhibitor from among the PD-1 immune checkpoint inhibitor and the PD-L1 immune checkpoint inhibitor and a group of responders (stable disease, SD) who showed a low effect in the anticancer treatment through at least one immune checkpoint inhibitor from among the PD-1 immune checkpoint inhibitor and the PD-L1 immune checkpoint inhibitor according to the protein measurement amounts of Complement Component 7 (hereinafter, referred to as 'Experimental Example 2').

### Experimental Example 3. When cutoff value for protein measurement amount of Complement Component C7 in companion diagnosis biomarker composition according to present invention is 110 µg/mL (comparison with PD-L1 pharmDx test)

In the same manner as in Experimental Example 1 except that a cutoff value of the protein amount measured for Complement Component C7 was set to 110 µg/mL, when the protein amount of Complement Component C7 was equal to or more than the cutoff value, samples were classified into a group of non-responders (progressive disease, PD) who did not show an effect in the anticancer treatment through at least one immune checkpoint inhibitor from among the PD-1 immune checkpoint inhibitor and the PD-L1 immune checkpoint inhibitor, and when the protein amount of Complement Component C7 was less than the cutoff value, samples were classified into a group of responders (partial response, PR) who showed a high effect in the anticancer treatment through at least one immune checkpoint inhibitor from among the PD-1 immune checkpoint inhibitor and the PD-L1 immune checkpoint inhibitor and a group of responders (stable disease, SD) who showed a low effect in the anticancer treatment through at least one immune checkpoint inhibitor from among the PD-1 immune checkpoint inhibitor and the PD-L1 immune checkpoint inhibitor according to the protein measurement amounts of Complement Component 7 (hereinafter, referred to as 'Experimental Example 3').

### Comparative Experimental Example 1. Companion diagnosis test through "PD-L1 pharmDx test"

A PD-L1 pharmDx test (hereinafter, referred to as 'pharmDx') is a companion diagnosis test used to determine whether pembrolizumab efficiently exhibits anticancer effects as an immune anticancer drug in specific patients.

Twenty seven tissue samples of the lung cancer patients in Experimental Example 1 were prepared, and the expression level of a programmed cell death protein 1 ligand (PD-L1) protein was observed by immunohistochemical staining of cancer cells in a biopsied tissue sample using a PD-L1 pharmDx product manufactured by DAKO Corporation to qualitatively test the PD-L1 protein. After the PD-L1 pharmDx test, a tumor proportion score (TPS), which is a percentage of effective tumor cells showing partial or entire cell membrane staining of tumor cells, was calculated by observing stained slides under an optical microscope, samples were classified into a group of responders (partial response, PR) who showed a high effect in the anticancer treatment through at least one immune checkpoint inhibitor from among the PD-1 immune checkpoint inhibitor and the PD-L1 immune checkpoint inhibitor when the TPS ≥ 50 %, samples were classified into a group of responders (stable disease, SD) who showed a low effect in the anticancer treatment through at least one immune checkpoint inhibitor from among the PD-1 immune checkpoint inhibitor and the PD-L1 immune checkpoint inhibitor when the TPS was 1 to 49%, and samples were classified into a group of non-responders (progressive disease, PD) who did not show an effect in the anticancer treatment through at least one immune checkpoint inhibitor from among the PD-1 immune checkpoint inhibitor and the PD-L1 immune checkpoint inhibitor when the TPS < 1 % (hereinafter, referred to as 'Comparative Experimental Example 1').

### Evaluation 4. Analysis and evaluation of results according to Experimental Examples 1 to 3 and Comparative Experimental Example 1

Hereinafter, the companion diagnosis test results according to the above-described Experimental Examples 1 to 3 and Comparative Experimental Example 1 were compared and evaluated.

The following Table 3 shows the results of the companion diagnosis tests according to Experimental Examples 1 to 3 and Comparative Experimental Example 1. In the following Table 3, "result" means the number of patients classified into the patient group (PR, PR+SD, PD) as a result of the corresponding experiment, and "actual" means the number of patients classified into the actual patient group (PR, PR+SD, PD) regardless of whether the corresponding experiment is carried out. That is, "result/actual" means a ratio of the number of patients classified into the patient group as a result of the corresponding experiment to the actual number of patients classified into the corresponding patient group.

**[Table 3]**

| | Comparative Experimental Example 1 | | Experimental Example 1 | | Experimental Example 2 | | Experimental Example 3 | |
|---|---|---|---|---|---|---|---|---|
| | Percentage (%) | result/actual | Percentage (%) | result/actual | Percentage (%) | result/actual | Percentage (%) | result/actual |
| PR | 33 | 2/6 | 100 | 6/6 | 100 | 6/6 | 100 | 6/6 |
| PR + SD | 38 | 5/13 | 85 | 11/13 | 92 | 12/13 | 100 | 13/13 |
| PD | 100 | 8/8 | 75 | 6/8 | 75 | 6/8 | 63 | 5/8 |

Referring to Table 3, it can be confirmed that in the case of Comparative Experimental Example 1 corresponding to the PD-L1 pharmDx test, the accuracy of determining a group of responders (partial response, PR) who show a high effect in the anticancer treatment through at least one immune checkpoint inhibitor from among the PD-1 immune checkpoint inhibitor and the PD-L1 immune checkpoint inhibitor and a group of responders (stable disease, SD) who show a low effect in the anticancer treatment through at least one immune checkpoint inhibitor from among the PD-1 immune checkpoint inhibitor and the PD-L1 immune checkpoint inhibitor is lower than Experimental Examples 1 to 3.

In contrast, it can be confirmed that in the case of Experimental Examples 1 to 3, as a result of performing a companion diagnosis test through the companion diagnosis biomarker composition according to the present invention, the accuracy of determining a group of non-responders (progressive disease, PD) tends to be relatively somewhat lower than that in Comparative Experimental Example 1, but the accuracy of determining a group of responders (partial response, PR) who show a high effect in the anticancer treatment through at least one immune checkpoint inhibitor from among the PD-1 immune checkpoint inhibitor and the PD-L1 immune checkpoint inhibitor and a group of responders (stable disease, SD) who show a low effect in the anticancer treatment through at least one immune checkpoint inhibitor from among the PD-1 immune checkpoint inhibitor and the PD-L1 immune checkpoint inhibitor is significantly higher than that in Comparative Experimental Example 1.

Therefore, it can be confirmed that in the case of Experimental Examples 1 to 3, the companion diagnosis test results through the companion diagnosis biomarker composition according to the present invention are a companion diagnosis test whose accuracy is better than in Comparative Experimental Example 1 corresponding to the PD-L1 pharmDx test.

### Experimental Example 4. When cutoff value for protein measurement amount of Complement Component C7 in companion diagnosis biomarker composition according to present invention is 100 µg/mL (comparison with SP263)

Thirty six blood samples of lung cancer patients were prepared, serum was extracted from each of the thirty six blood samples, and then subjected to a pre-treatment process, and each of the protein amounts for Complement Component C7 was measured by performing proteomic analysis on the thirty six samples of serum using TMT10 isobaric compounds. However, here, in the protein analysis, each of the protein amounts for Complement Component C7 was measured by quantitative analysis.

In this case, the cutoff value of the protein amount measured for Complement Component C7 was set to 100 µg/mL and when the protein amount of Complement Component C7 was equal to or more than a cutoff value, samples were classified into a group of non-responders (progressive disease, PD) who did not show an effect in the anticancer treatment through at least one immune checkpoint inhibitor from among the PD-1 immune checkpoint inhibitor and the PD-L1 immune checkpoint inhibitor, and when the protein amount of Complement Component C7 was less than the cutoff value, samples were classified into a group of responders (partial response, PR) who showed a high effect in the anticancer treatment through at least one immune checkpoint inhibitor from among the PD-1 immune checkpoint inhibitor and the PD-L1 immune checkpoint inhibitor and a group of responders (stable disease, SD) who showed a low effect in the anticancer treatment through at least one immune checkpoint inhibitor from among the PD-1 immune checkpoint inhibitor and the PD-L1 immune checkpoint inhibitor according to the protein measurement amounts of Complement Component 7 (hereinafter, referred to as 'Experimental Example 4').

### Experimental Example 5. When cutoff value for protein measurement amount of Complement Component C7 in companion diagnosis biomarker composition according to present invention is 105 µg/mL (comparison with SP263)

In the same manner as in Experimental Example 4 except that a cutoff value of the protein amount measured for Complement Component C7 was set to 105 µg/mL, when the protein amount of Complement Component C7 was equal to or more than the cutoff value, samples were classified into a group of non-responders (progressive disease, PD) who did not show an effect in the anticancer treatment through at least one immune checkpoint inhibitor from among the PD-1 immune checkpoint inhibitor and the PD-L1 immune checkpoint inhibitor, and when the protein amount of Complement Component C7 was less than the cutoff value, samples were classified into a group of responders (partial response, PR) who showed a high effect in the anticancer treatment through at least one immune checkpoint inhibitor from among the PD-1 immune checkpoint inhibitor and the PD-L1 immune checkpoint inhibitor and a group of responders (stable disease, SD) who showed a low effect in the anticancer treatment through at least one immune checkpoint inhibitor from among the PD-1 immune checkpoint inhibitor and the PD-L1 immune checkpoint inhibitor according to the protein measurement amounts of Complement Component 7 (hereinafter, referred to as 'Experimental Example 5').

### Experimental Example 6. When cutoff value for protein measurement amount of Complement Component C7 in companion diagnosis biomarker composition according to present invention is 110 µg/mL (comparison with SP263)

In the same manner as in Experimental Example 4 except that a cutoff value of the protein amount measured for Complement Component C7 was set to 110 µg/mL, when the protein amount of Complement Component C7 was equal to or more than the cutoff value, samples were classified into a group of non-responders (progressive disease, PD) who did not show an effect in the anticancer treatment through at least one immune checkpoint inhibitor from among the PD-1 immune checkpoint inhibitor and the PD-L1 immune checkpoint inhibitor, and when the protein amount of Complement Component C7 was less than the cutoff value, samples were classified into a group of responders (partial response, PR) who showed a high effect in the anticancer treatment through at least one immune checkpoint inhibitor from among the PD-1 immune checkpoint inhibitor and the PD-L1 immune checkpoint inhibitor and a group of responders (stable disease, SD) who showed a low effect in the anticancer treatment through at least one immune checkpoint inhibitor from among the PD-1 immune checkpoint inhibitor and the PD-L1 immune checkpoint inhibitor according to the protein measurement amounts of Complement Component 7 (hereinafter, referred to as 'Experimental Example 6').

### Comparative Experimental Example 2. Companion diagnosis test through "VENTANA PD-L1 (SP263) assay

A VENTANA PD-L1 (SP263) assay (hereinafter, referred to as 'SP263') is a companion diagnosis test used to determine whether nivolumab efficiently exhibits anticancer effects as an immune anticancer drug in specific patients.

Thirty six tissue samples of the lung cancer patients in Experimental Example 1 were prepared, and the expression level of a programmed cell death protein 1 ligand (PD-L1) protein was observed by immunohistochemical staining of cancer cells in a biopsied tissue sample using a VENTANA PD-L1 (SP263) assay product to qualitatively test the PD-L1 protein. After the PD-L1 pharmDx test, a tumor proportion score (TPS), which is a percentage of effective tumor cells showing partial or entire cell membrane staining of tumor cells, was calculated by observing stained slides under an optical microscope, samples were classified into a group of responders (partial response, PR) who showed a high effect in the anticancer treatment through at least one immune checkpoint inhibitor from among the PD-1 immune checkpoint inhibitor and the PD-L1 immune checkpoint inhibitor when the TPS ≥ 10 %, samples were classified into a group of responders (stable disease, SD) who showed a low effect in the anticancer treatment through at least one immune checkpoint inhibitor from among the PD-1 immune checkpoint inhibitor and the PD-L1 immune checkpoint inhibitor when the TPS was 1 to 9%, and samples were classified into a group of non-responders (progressive disease, PD) who did not show an effect in the anticancer treatment through at least one immune checkpoint inhibitor from among the PD-1 immune checkpoint inhibitor and the PD-L1 immune checkpoint inhibitor when the TPS < 1 % (hereinafter, referred to as 'Comparative Experimental Example 2').

### Evaluation 5. Analysis and evaluation of results according to Experimental Examples 4 to 6 and Comparative Example 2

Hereinafter, the companion diagnosis test results according to the above-described Experimental Examples 4 to 6 and Comparative Experimental Example 2 were compared and evaluated.

The following Table 4 shows the results of the companion diagnosis tests according to Experimental Examples 4 to 6 and Comparative Experimental Example 2. In the following Table 4, "result" means the number of patients classified into the patient group (PR, PR+SD, PD) as a result of the corresponding experiment, and "actual" means the number of patients classified into the actual patient group (PR, PR+SD, PD) regardless of whether the corresponding experiment is carried out. That is, "result/actual" means a ratio of the number of patients classified into the patient group as a result of the corresponding experiment to the actual number of patients classified into the corresponding patient group.

**[Table 4]**

| | Comparative Experimental Example 2 | | Experimental Example 4 | | Experimental Example 5 | | Experimental Example 6 | |
|---|---|---|---|---|---|---|---|---|
| | Percentage (%) | result/actual | Percentage (%) | result/actual | Percentage (%) | result/actual | Percentage (%) | result/actual |
| PR | 67 | 6/9 | 100 | 9/9 | 100 | 9/9 | 100 | 9/9 |
| PR + SD | 71 | 12/17 | 76 | 13/17 | 88 | 15/17 | 100 | 17/17 |
| PD | 80 | 8/10 | 90 | 9/10 | 90 | 9/10 | 70 | 7/10 |

Referring to Table 4, it can be confirmed that in the case of Comparative Experimental Example 2 corresponding to the VENTANA PD-L1 (SP263) assay, the accuracy of determining a group of responders (partial response, PR) who show a high effect in the anticancer treatment through at least one immune checkpoint inhibitor from among the PD-1 immune checkpoint inhibitor and the PD-L1 immune checkpoint inhibitor and a group of responders (stable disease, SD) who show a low effect in the anticancer treatment through at least one immune checkpoint inhibitor from among the PD-1 immune checkpoint inhibitor and the PD-L1 immune checkpoint inhibitor is lower than Experimental Examples 4 to 6. In addition, it can be confirmed that the accuracy of determining a group of non-responders (progressive disease, PD) who do not show an effect in the anticancer treatment through at least one immune checkpoint inhibitor from among the PD-1 immune checkpoint inhibitor and the PD-L1 immune checkpoint inhibitor is also relatively lower than in Experimental Examples 4 to 6.

In contrast, it can be confirmed that in the case of Experimental Examples 4 to 6, as a result of performing a companion diagnosis test through the companion diagnosis biomarker composition according to the present invention, the accuracy of determining a group of responders (partial response, PR) who show a high effect in the anticancer treatment through at least one immune checkpoint inhibitor from among the PD-1 immune checkpoint inhibitor and the PD-L1 immune checkpoint inhibitor, a group of responders (stable disease, SD) who show a low effect in the anticancer treatment through at least one immune checkpoint inhibitor from among the PD-1 immune checkpoint inhibitor and the PD-L1 immune checkpoint inhibitor, and a group of non-responders (progressive disease, PD) who do not show an effect in the anticancer treatment through at least one immune checkpoint inhibitor from among the PD-1 immune checkpoint inhibitor and the PD-L1 immune checkpoint inhibitor is relatively higher than that in Comparative Experimental Example 2.

Therefore, it can be confirmed that in the case of Experimental Examples 4 to 6, the companion diagnosis test results through the companion diagnosis biomarker composition according to the present invention are a companion diagnosis test whose accuracy is better than in Comparative Experimental Example 2 corresponding to the VENTANAPD-L1 (SP263) assay.

The companion diagnosis biomarker composition of the present invention as described above and the companion diagnosis kit containing the same have an effect that it is possible to predict a therapeutic response to at least one immune checkpoint inhibitor from among the PD-1 immune checkpoint inhibitor and the PD-L1 immune checkpoint inhibitor not only through a companion diagnosis through cancer patient tissues, but also through proteomic analysis of cancer patient blood.

In addition, the companion diagnosis biomarker composition according to the present invention and the companion diagnosis kit containing the same have an advantage in that the accuracy of screening a group of responders who show an effect in anticancer treatment through at least one immune checkpoint inhibitor from among a PD-1 immune checkpoint inhibitor and a PD-L1 immune checkpoint inhibitor and a group of non-responders who do not show an effect in the anticancer treatment.

Furthermore, the companion diagnosis biomarker composition according to the present invention and the companion diagnosis kit containing the same have an effect that it is possible to more accurately determine a group of patients suitable for anticancer treatment through at least one immune checkpoint inhibitor from among a PD-1 immune checkpoint inhibitor and a PD-L1 immune checkpoint inhibitor.

While the aforementioned description merely exemplarily describes the technical spirit of the present invention, a person with ordinary skill in the art to which the present invention pertains can make various modifications and alterations within the scope not departing from the essential characteristics of the present invention.

Accordingly, the embodiments disclosed in the present invention are intended not to limit but to describe the technical spirit of the present invention, and the scope of the technical spirit of the present invention is not limited by these embodiments. The protection scope of the present invention must be interpreted by the following claims and it should be interpreted that all technical ideas within a scope equivalent thereto are included in the scope of rights of the present invention.

## Claims

1. A companion diagnosis biomarker composition comprising Complement Component C7, wherein the companion diagnosis biomarker composition predicts the reactivity of at least one immune checkpoint inhibitor from among a programmed cell death protein 1 (PD-1) immune checkpoint inhibitor and a programmed death-ligand 1 (PD-L1) immune checkpoint inhibitor against cancer cells.

2. The companion diagnosis biomarker composition of claim 1, further comprising one or more biomarkers selected from the group consisting of a histidinerich glycoprotein (HRG), a zinc-alpha-2-glycoprotein (AZGP1), Complement Component C5, immunoglobulin kappa variable 4-1 (IHKV4-1), mannosyloligosaccharide 1,2-alpha-mannosidase 1A (MANIA), alpha-2-macroglobulin (A2M), osteopontin (SPP1) and hypoxia up-regulated protein 1 (HYOU1).

3. The companion diagnosis biomarker composition of claim 1, wherein a protein amount of Complement Component C7 is measured by quantitative analysis.

4. The companion diagnosis biomarker composition of claim 1, wherein a protein amount of Complement Component C7 is measured by any one method selected from the group consisting of protein mass analysis, protein chip analysis, an immune measurement method, a ligand binding assay, matrix desorption/ionization time of flight mass spectrometry (MALDI-TOF) analysis, surface enhanced laser desorption/ionization time of flight mass spectrometry (SELDI-TOF) analysis, a radioimmunoassay, a radial immunodiffusion method, an Ouchterlony immunodiffusion method, rocket immunoelectrophoresis, tissue immunostaining, a complement fixation assay method, 2-dimensional electrophoresis analysis, liquid chromatography-mass spectrometry (LC-MS), liquid chromatography-mass spectrometry/mass spectrometry (LC-MS/MS), western blot and enzyme linked immunosorbent assay (ELISA).

5. The companion diagnosis biomarker composition of claim 1, wherein for Complement Component C7, the amount of protein is decreased in a group of responders who show an effect in anticancer treatment through the PD-1 immune checkpoint inhibitor compared to a group of non-responders who do not show an effect in the anticancer treatment through the PD-1 immune checkpoint inhibitor.

6. The companion diagnosis biomarker composition of claim 1, wherein for Complement Component C7, the amount of protein is increased in a group of non-responders who do not show an effect in anticancer treatment through the PD-1 immune checkpoint inhibitor compared to a group of responders who show an effect in the anticancer treatment through the PD-1 immune checkpoint inhibitor.

7. The companion diagnosis biomarker composition of claim 1, wherein for Complement Component C7, the amount of protein is decreased in a group of responders who show an effect in anticancer treatment through the PD-L1 immune checkpoint inhibitor compared to a group of non-responders who do not show an effect in the anticancer treatment through the PD-L1 immune checkpoint inhibitor.

8. The companion diagnosis biomarker composition of claim 1, wherein for Complement Component C7, the amount of protein is increased in a group of non-responders who do not show an effect in anticancer treatment through the PD-L1 immune checkpoint inhibitor compared to a group of responders who show an effect in the anticancer treatment through the PD-L1 immune checkpoint inhibitor.

9. The companion diagnosis biomarker composition of claim 1, wherein a cutoff value for a protein amount of Complement Component C7 is in a range of 100 to 110 |ig/mL,

10. The companion diagnosis biomarker composition of claim 9, wherein when the protein amount of Complement Component C7 is equal to or more than the cutoff value, the responsiveness to the PD-1 immune checkpoint inhibitor for the cancer cells is low.

11. The companion diagnosis biomarker composition of claim 9, wherein when the protein amount of Complement Component C7 is less than the cutoff value, the responsiveness to the PD-1 immune checkpoint inhibitor for the cancer cells is high.

12. The companion diagnosis biomarker composition of claim 9, wherein when the protein amount of Complement Component C7 is equal to or more than the cutoff value, the responsiveness to the PD-L1 immune checkpoint inhibitor for the cancer cells is low.

13. The companion diagnosis biomarker composition of claim 9, wherein when the protein amount of Complement Component C7 is less than the cutoff value, the responsiveness to the PD-L1 immune checkpoint inhibitor for the cancer cells is high.

14. The companion diagnosis biomarker composition of claim 1, wherein the companion diagnosis biomarker composition is extracted from any one selected from the group consisting of blood, serum, plasma and tissue.

15. The companion diagnosis biomarker composition of claim 14, wherein the companion diagnosis biomarker composition is extracted from blood.

16. The companion diagnosis biomarker composition of claim 14, wherein the companion diagnosis biomarker composition is extracted from tissue.

17. The companion diagnosis biomarker composition of claim 16, further comprising a programmed death-ligand (PD-L1) protein.

18. The companion diagnosis biomarker composition of claim 1, wherein the cancer cells are cancer cells corresponding to carcinomas specifically responding to at least one immune checkpoint inhibitor from among the PD-1 immune checkpoint inhibitor and the PD-L1 immune checkpoint inhibitor.

19. The companion diagnosis biomarker composition of claim 1, wherein the cancer cells are cancer cells corresponding to any one carcinoma selected from the group consisting of lung cancer, liver cancer, gastric cancer, gastric and gastroesophageal junction adenocarcinoma, skin melanoma, head and neck cancer, bone cancer, pancreatic cancer, skin cancer, uterine cancer, ovarian cancer, rectal cancer, colorectal cancer, colon cancer, breast cancer, uterine sarcoma, fallopian tube carcinoma, endometrial carcinoma, cervical carcinoma, vaginal carcinoma, vulvar carcinoma, esophageal cancer, laryngeal cancer, small intestine cancer, thyroid cancer, parathyroid cancer, soft tissue sarcoma, urethral cancer, penile cancer, prostate cancer, chronic or acute leukemia, solid tumors of childhood, differentiated lymphoma, bladder cancer, kidney cancer, renal cell carcinoma, renal pelvic carcinoma, primary central nervous system lymphoma, spinal cord tumors, brainstem glioma and pituitary adenoma.

20. The companion diagnosis biomarker composition of claim 19, wherein the cancer cells are cancer cells corresponding to lung cancer or liver cancer.

21. The companion diagnosis biomarker composition of claim 1, wherein the companion diagnosis biomarker composition is administered simultaneously or sequentially in combination with at least one immune checkpoint inhibitor from among the PD-1 immune checkpoint inhibitor and the PD-L1 immune checkpoint inhibitor.

22. A companion diagnosis kit comprising the companion diagnosis biomarker composition according to any one of claims 1 to 21.
